# EUROPEAN PATENT APPLICATION

(11) **EP 4 155 733 A1**
(43) Date of publication of application: **29.03.2023**
(21) Application number: 22172530.2
(22) Date of filing: 10.05.2022
(51) Int. Cl.: G01N 33/48, C07K 14/47

(54) **MOUSE MODELS FOR MULTIPLE SYSTEM ATROPHY**

(30) Priority: 22.09.2021 EP 21198225
(71) Applicant: Katholieke Universiteit Leuven, KU Leuven R&D, 3000 Leuven (BE)
(72) Inventor: The designation of the inventor has not yet been filed

(57) **Abstract**

The invention relates to non-therapeutic methods of screening a candidate compound for use as a drug for the treatment or prevention of a demyelinating disease using transgenic rodent wherein the expression of wild type rodent alpha synuclein is inactivated and carrying a transgenic construct expressing human alpha synuclein in neurons and oligodendrocytes.

## Description

### FIELD OF THE INVENTION

The invention describes a mouse model wherein peripheral environmental triggers result in pathology reminiscent of multiple system atrophy.

### INTRODUCTION

Multiple system atrophy (MSA) is a rare and fatal neurodegenerative disease of unknown origin. It is believed that MSA risk is influenced by genetic and environmental factors [Palma et al. Auton. Neurosci. 211, 15-25 (2018); Fanciulli et al. N. Engl. J. Med. 372, 249-263 (2015)] but the disease is relatively rare and no strong risk factors have been identified so far. MSA progressively affects a wide variety of motor, cognitive and autonomous nervous system functions, but it invariably leads to severe disability and death [Jellinger Free Neuropathology 1, 17 (2020)]. Some of the earliest features in MSA include erectile and urinary bladder dysfunction, which can debut several years before the onset of motor symptoms [Kirchhof et al. Int. J. Impot. Res. 15, 293-298 (2003)]. It is estimated that over 20% of MSA patients experience lower urogenital symptoms as their initial complaint [Sakakibara et al. Clin. Auton. Res., 1-5 (2018); Sakakibara et al. J. Neurol. Neurosurg. Psychiatry 68, 65-69 (2000)], suggesting that in some cases the MSA disease process might start in the urogenital system [Panicker et al. J. Neurol. 267, 659-664 (2019)].

αSyn is a membrane binding protein that favourably associates with small vesicular organelles in neurons of the central and peripheral nervous systems [Bendor et al. Neuron 79, 1044-1066 (2013); Lautenschläger et al. Trends Cell Biol. 27, 468-479 (2017)]. The presence of aggregated α-synuclein (αSyn) in brain oligodendrocytes is a pathognomonic feature of MSA [Papp et al. J. Neurol. Sci. 94, 79-100. (1989); Spillantini et al. Neurosci. Lett. 25, 205-208 (1998)]. Neuronal inclusions of αSyn are also observed in MSA, but to a lesser extent. The distribution of pathology in the central nervous system (CNS) varies and two main cluster have been identified. They are MSA-P with a predominance of basal ganglia pathology and clinical parkinsonism; and MSA-C with synucleinopathy in cerebellar and brainstem circuits connected to the spinal cord, presenting clinically with ataxia [Halliday et al. Acta Neuropathol 122, 187-204 (2011); Wenning et al. Brain 117, 835-845 (1994)]. Oligodendrocytes of the spinal cord and the brain normally express low levels of aSyn [Djelloul et al. Stem Cell Reports 5, 174-184 (2015); Asi et al. Glia 62, 964-970 (2014)] but some oligodendrocytes can express high levels of αSyn during inflammation and demyelination [Falcão et al. Nat. Med. 24, 1837-1844 (2018); Jäkel et al. Nature 566, 543-547 (2019)]. Aggregation of αSyn in oligodendrocytes leads to the assembly of conformation-specific αSyn fibrils with unique and highly aggregation-prone and pathogenic features [Peng et al. Nature 557, 558-563 (2018)]. In MSA patients, aggregates of αSyn are also detected in Schwann cells of the spinal nerves and in neurons of visceral organs, including the urinary bladder [Ding et al. iScience 23, 101166 (2020); Nakamura Acta Neuropathol. Commun. 3, art29 (2015)]. Transgenic MSA mice develop αSyn inclusions in the urinary bladder [Boudes et al. Mov. Disord. 28, 347-355 (2013)], and recently it was shown that aggregates of αSyn injected in the lower urinary tract can result in propagation of αSyn aggregates to the spinal cord in mice.

Synucleinopathy can be triggered peripherally by a pathogen [Tulisiak et al. in Molecular Biology of Neurodegenerative Diseases: Visions for the Future, Part A, (Elsevier, 2019), pp. 299-322] and the disease trigger can occur before or during advanced disease stages and elicit a local or systemic host response. The idea that αSyn might participate in the regulation of immune responses has been proposed recently [Labrie & Brundin J. Innate Immun. 9, 437-440 (2017)], but its precise role in that context remains elusive. Urinary tract infections (UTIs) are among the most common bacterial infections in humans [Foxman Nat. Rev. Urol 7, 653-660 (2010)] and are prominent in MSA, both before and after formal diagnosis of MSA [Papapetropoulos et al. J. Neurol. Neurosurg. Psychiatry 78, 327 (2006); Papatsoris et al. Neurourol. Urodyn. 27, 22-27 (2007)]. It is estimated that more than half of MSA patients suffer from recurrent or chronic UTIs, which can lead to urosepsis and death.

Given the high frequency of UTIs in prodromal MSA, we investigated if synucleinopathy could be triggered by bacterial infections in the urinary tract, and if injections of αSyn aggregates from MSA patients into the urinary bladder of mice can propagate to the CNS giving rise to oligodendroglial pathology and motor deficits. We find that deposits of αSyn are present in the human urinary bladder wall of normal subjects and MSA patients. We show that in mice uropathogenic E. *coli* (UPEC) cause infiltration of neutrophils that express and release αSyn. Sarkosyl extraction of αSyn from infected urinary bladder shows that αSyn forms insoluble aggregates. In a cell culture model, we observe in real time that αSyn is released via extracellular traps (NETs) upon stimulation by UPEC. Finally, following injection of αSyn aggregates derived from MSA patients into the urinary bladder of humanized αSyn transgenic mice, bladder dysfunction, motor deficits with oligodendroglial αSyn pathology all progressively develop in the CNS resulting in an animal model which mimics key features of MSA.

### Summary of the invention

Symptoms in the urogenital organs, as well as urinary tract infections, are common in multiple system atrophy (MSA), also in the years preceding the MSA diagnosis. It is unknown how MSA is triggered and these observations in prodromal MSA indicate that synucleinopathy could be triggered by bacterial infection of the genitourinary tract causing α-synuclein (αSyn) to aggregate in peripheral nerves innervating these organs. Here we show that bacterial infection of the urinary bladder can cause synucleinopathy in mice and we propose a novel role of αSyn in the innate immune system response to bacteria. Urinary tract infection with uropathogenic E. *coli* results in the *de novo* aggregation of αSyn during neutrophil infiltration. We show that during the infection αSyn is released extracellularly from neutrophils as part of their extracellular traps. We demonstrate that injection of MSA aggregates into the urinary bladder leads to motor deficits and propagates αSyn pathology to the central nervous system in mice overexpressing αSyn. Our results link bacterial infections with synucleinopathy and show that a host response to environmental triggers can result in αSyn pathology that bears semblance to MSA.

The present method provides a rodent model for MSA by inactivating the rodent alpha synuclein and introducing a transgene expression human alpha synuclein. This model develops an expression pattern of human synuclein which is highly similar to the expression of human synuclein as encountered in human MSA patients.

It is envisaged that such model provides a more reliable screening of test compounds for MSA.

In addition of being suitable in treating and preventing MSA, the candidate compounds as identified by the methods of the present invention are likely candidates as well for other demyelinating diseases such as Multiple Sclerosis.

The invention is further summarised in the following statements.
1. A non-therapeutic method of screening a candidate compound for use as a drug for the treatment or prevention of a demyelinating disease, comprising the steps of:
   a) Providing a transgenic rodent wherein the expression of wild type rodent alpha synuclein is inactivated and carrying a transgenic construct expressing human alpha synuclein in neurons and oligodendrocytes,
   b) delivering to the transgenic rodent of step a candidate compound,
   c) determining the expression of alpha synuclein in oligodendrocytes and/or of the rodent, and comparing with the expression in a control rodent as described in step a) which did not receive the candidate compound,
   wherein a decrease in alpha synuclein expressed in oligodendrocytes compared to the control rodent is indicative of a therapeutic activity of the candidate compound against the demyelinating disease.
2. The method according to statement 1, wherein the demyelinating disease is Multiple System Atrophy (MSA).
3. The method according to statement 1 or 2, wherein the rodent is sacrificed prior to determining the expression of alpha synuclein in step c).
4. The method according to any one of statements 1 to 3, wherein the expression of wild type rodent alpha synuclein is inactivated by deletion of the wild type alpha synuclein gene.
5. The method according to any one of statements 1 to 4, further determining the expression of human alpha synuclein in the central and/or peripheral nervous system, wherein a decrease in alpha synuclein expressed in central and/or peripheral nervous system compared to the control rodent is indicative of a therapeutic activity of the test compound against the demyelinating disease.
6. The method according to any one of statements 1 to 5, further delivering to the rodent animal human alpha synuclein via viral vectors and/or injection of alpha synuclein fibrils.
7. The method according to any one of statements 1 to 6, wherein the test compound is administered prior to the development of MSA pathology.
8. The method according to any one of statements 1 to 7, wherein the test compound is administered to a rodent showing MSA pathology.
9. The method according to any one of statements 1 to 8, wherein the expression of the human alpha synuclein is under the control of human alpha synuclein promotor.
10. The method according to any one of statements 1 to 9, wherein the human alpha synuclein is wild type alpha synuclein.
11. The method according to any one of statements 1 to 10, wherein the test compound is a recombinant Adeno Associated viral vector comprising miRNA against alpha synuclein.

### DESCRIPTION OF THE INVENTION

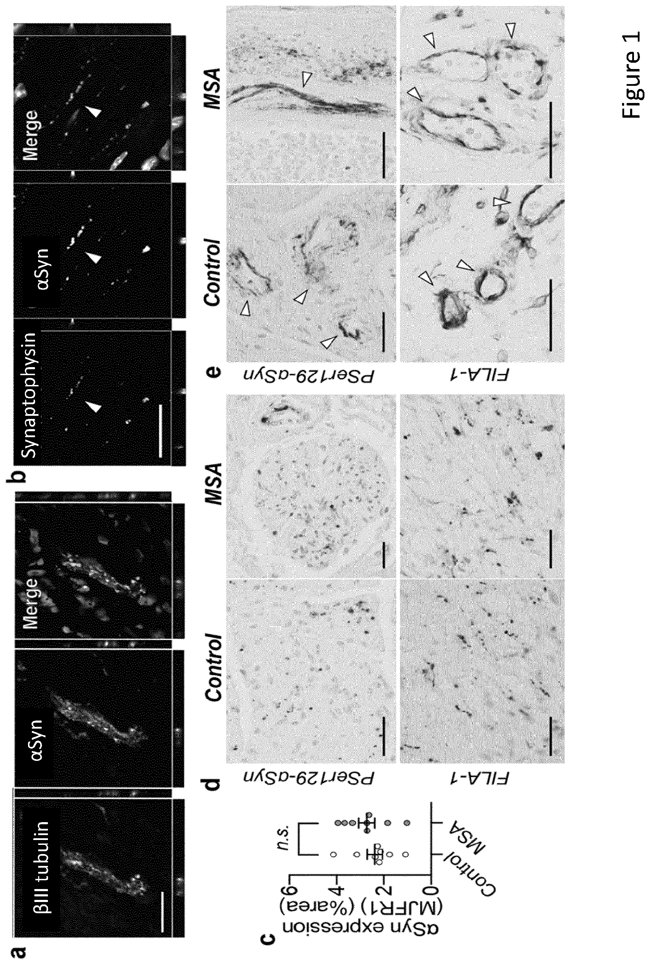

### Figure 1. Detection of pathological αSyn in the urinary bladder of MSA patients and controls.

Immunohistochemical analysis of αSyn in human urinary bladder shows αSyn expression in **(A)** βIII tubulin-positive intramural ganglia and **(B)** neuronal synapses. **(C)** αSyn expression in urinary bladder of MSA patients quantified in is not different from αSyn levels in urinary bladder of unaffected individuals (n=8, p > 0.05 with unpaired two-tailed t-test). **(D)** Detection of PSer129-αSyn (upper panel) and FILA-1 (lower panel) shows the presence of pathological αSyn in urinary bladder of controls and MSA cases. **(E)** Pathological αSyn (PSer129-αSyn and FILA-1) is also found around blood vessels (white arrows) in urinary bladder of normal subjects and MSA cases (scale bars represent 50 µm).

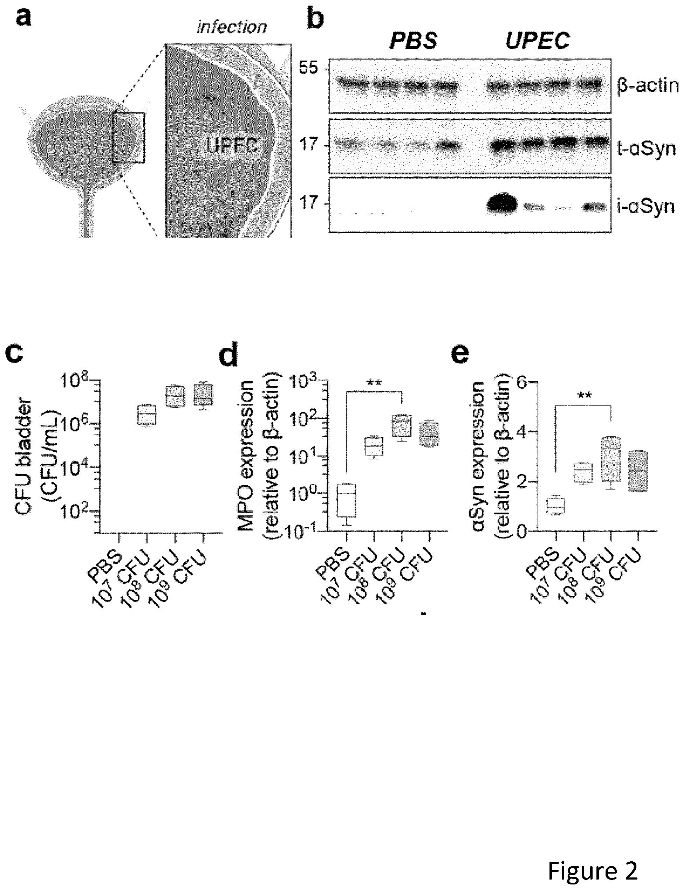

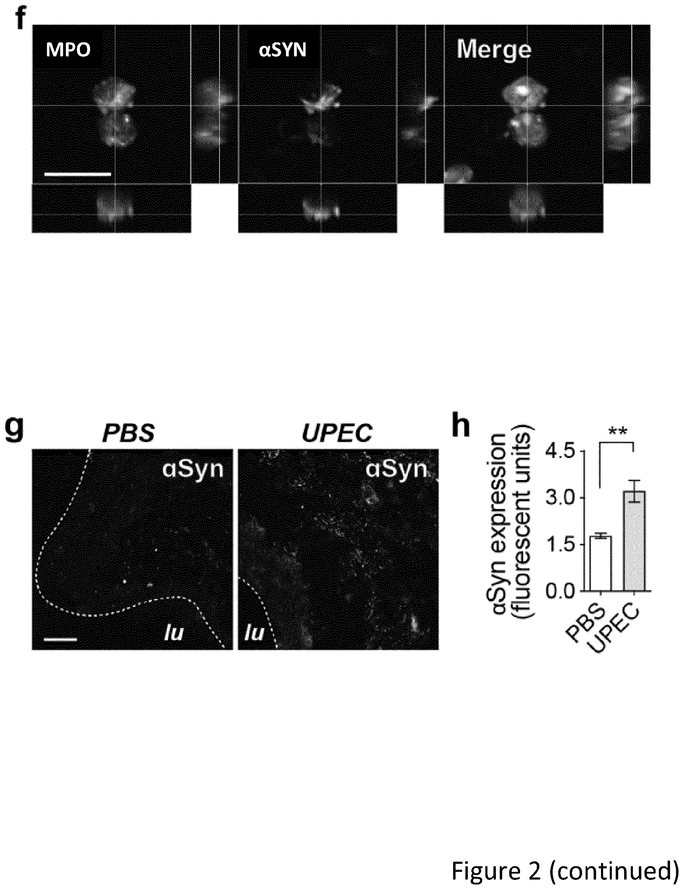

### Figure 2. Urinary tract infection triggers endogenous αSyn aggregation in vivo.

**(A)** Injection of 10⁸ colony forming units (CFU) of uropathogenic E. coli (UPEC) in the urinary bladder of C57BL/6N mice leads to **(B)** acute infection with **(C)** increased expression of αSyn after 12 hours of infection (n=4, ^{∗∗}p<0.01 with one-way ANOVA and Bonferroni post hoc correction for multiple testing). **(D)** Isolation of insoluble αSyn (i-aSyn) after sarkosyl treatment of whole urinary bladder homogenates reveals that αSyn is aggregated during infection. **(E)** The expression of αSyn coincides with the detection of myeloperoxidase (MPO) indicative of neutrophil infiltration (n=4, ^{∗∗}p<0.01 with Kruskal Wallis test and Dunn's post hoc correction for multiple testing), **(F)** Confocal Z-stack images of infected mouse urinary bladder shows that neutrophils infiltrating the infected tissue express αSyn during infection (scale bar 30 µm). **(G)** αSyn is detected in the lamina propria under basal conditions (lu, lumen, scale bar 50 µm) and increases significantly during infection with UPEC quantified in **(H)** (n=4, ^{∗∗}p<0.01 with one-way ANOVA and Bonferroni post hoc correction for multiple testing).

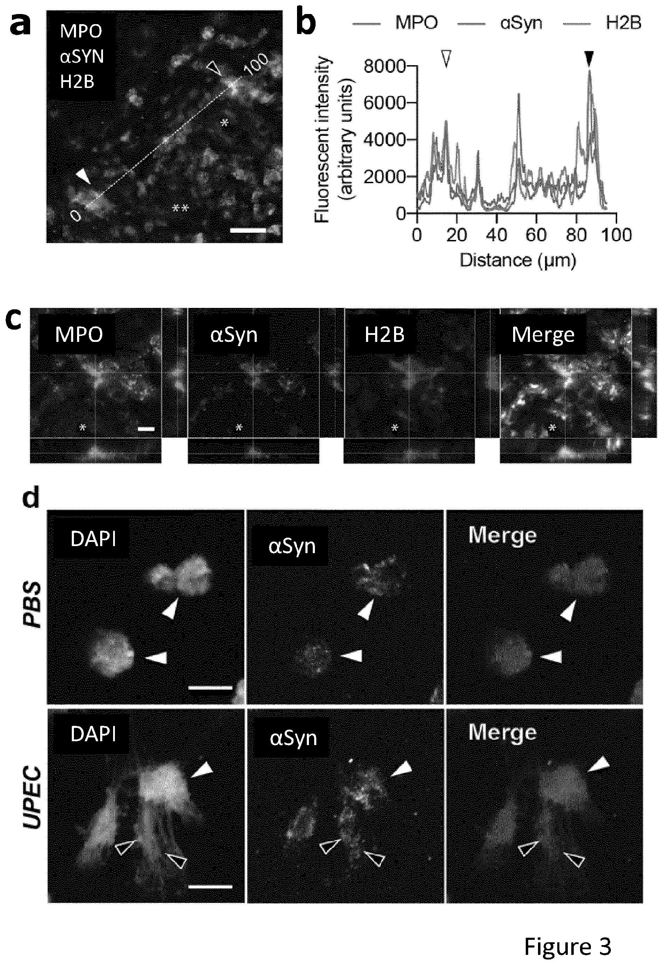

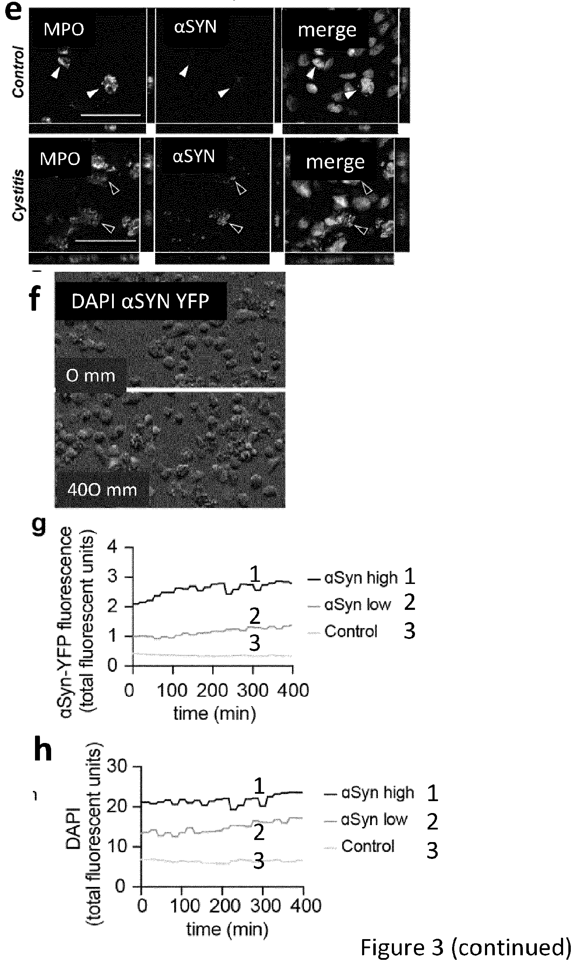

### Figure 3. αSyn is found extracellularly in neutrophil extracellular traps.

**(A)** Infected urinary bladders at 12 hours show NETs with αSyn in the vicinity of red blood vessels in the lamina propria (scale bar is 15 µm, ^{∗} and **indicate two blood vessels). **(B)** Intensity plot of MPO, H2B and αSyn along a 100 µm line in (A) shows overlap between the three markers. Highly αSyn expressing areas with strong intensity peaks are observed in NETs (open and closed arrows). **(C)** Confocal z-stacks of the NET in **(A)** (closed arrow) shows colocalization between MPO, αSyn and H2B (scale bar is 5 µm). **(D)** Primary human neutrophils express αSyn (white arrows), which is released via extracellular traps (black arrows) during infection with UPEC (10:1 MOI, scale bar is 20 µm). **(E)** In normal human urinary bladder neutrophils express relative low levels of αSyn while in a case of cystitis neutrophils with decondensing DNA release αSyn extracellularly (black arrows, scale bar is 40 µm). **(F)** The detection and release of extracellular DNA was visualized via live-imaging in aSyn-YFP expressing dHL-60 stimulated with UPEC (10:1 MOI). Control cells are WT dHL60 cells that do not express αSyn. **(G)** After infection control cells show background detection of fluorescent signal. αSyn-YFP low and high expressing cells show increasing fluorescent aSyn-YFP with **(H)** increased detection of aSyn-YFP in extracellular traps (experiment was performed in triplicate and showing one representative experiment)

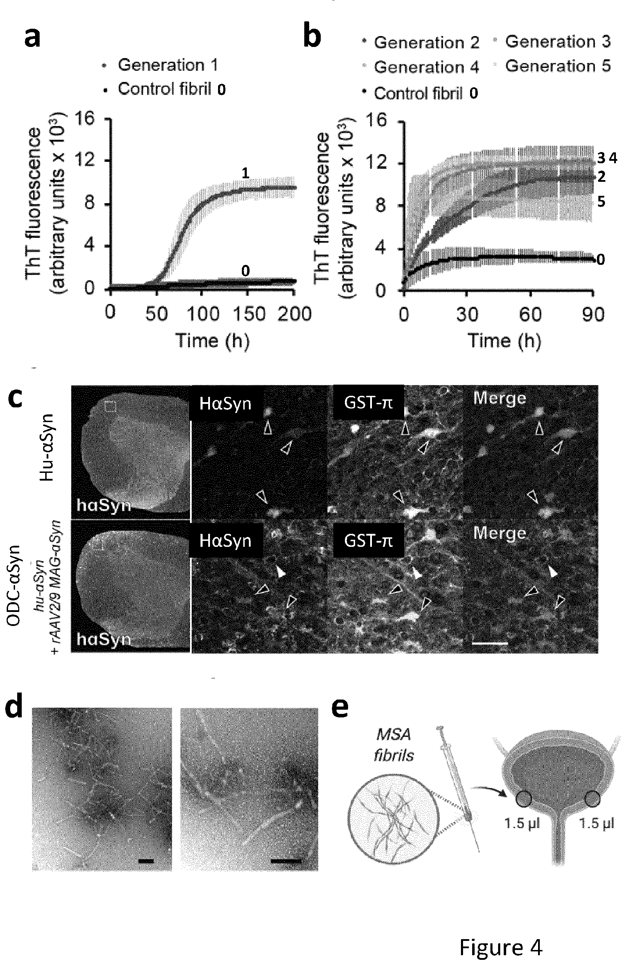

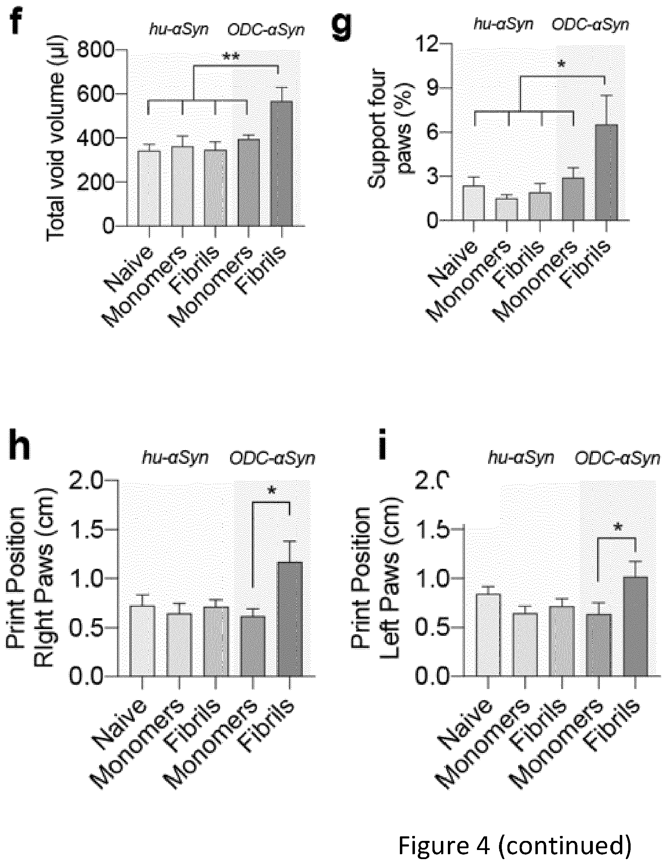

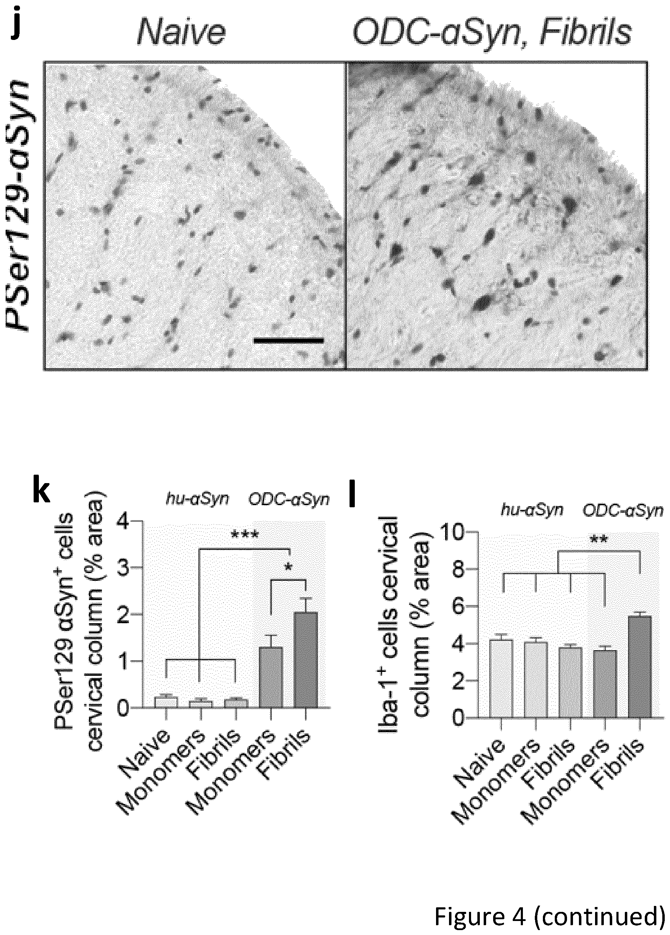

### Figure 4. MSA fibrils propagate synucleinopathy from the urinary tract in vivo.

To assess peripheral αSyn propagation and how oligodendroglial cellular environment can impact the spreading of aSyn, αSyn fibrils were amplified from human MSA brain. **(A)** After addition of homogenized and purified MSA brain, αSyn monomers were efficiently seeded into fibrillar αSyn. **(B)** Different passages with 5 rounds of amplification generates distinctive MSA fibrils with a distinct ThT profile compared to fibrils generated with recombinant αSyn only. **(C)** Two different animal models are used. Both models express human αSyn in spinal cord oligodendrocytes but with differences in expression pattern. Transgenic human αSyn mice (hu-aSyn mice), express soluble human αSyn in cell bodies of oligodendrocytes of the spinal cord (open arrows, upper panel) whereas ODC-aSyn mice express human αSyn in cell bodies and myelin sheets of mature oligodendrocytes in spinal cord anterior columns (lower panel, closed arrows). **(D)** Representative images of amplified fibrils of MSA brain recorded by TEM (scale bar = 100nm). **(E)** Fibrils from MSA brain were injected in two opposite sides of the detrusor muscle of the urinary bladder adjacent to the urethral opening and close the pelvic plexus. After 9 months of injection, animals showed **(F)** urinary voiding and **(G)** gait deficits with loss of fine motor control of **(H)** right and **(I)** left paws for ODC-aSyn mice injected with MSA fibrils but not with monomers (n≥6, ^{∗}p<0.05, ^{∗∗}p<0.01 with mixed-effects analysis of two-way ANOVA and Tukey post hoc correction for multiple comparison). No effects were observed in hu-aSyn mice injected with MSA fibrils. **(J)** Analysis of PSer129-αSyn in spinal cord reveals that in ODC-aSyn mice injection of MSA strains into the urinary bladder leads to. **(K)** increased αSyn pathology accompanied by **(L)** microglial inflammation (n≥6, s.e.m. ^{∗}p<0.05, ^{∗∗∗}p<0.001 with mixed-effects analysis of two-way ANOVA and Tukey post hoc correction for multiple comparison).

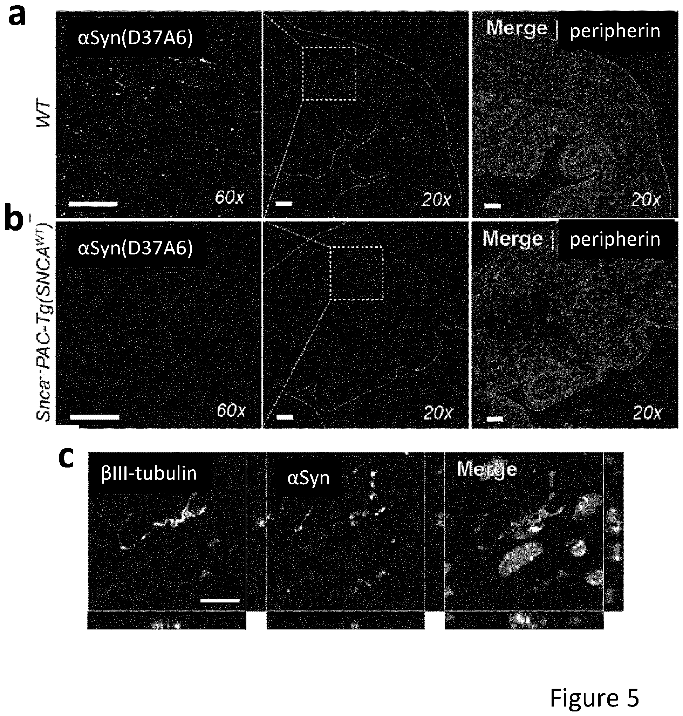

### Figure 5. αSyn expression in urinary bladder of WT mice.

Coronal sections of urinary bladder of C57BL/6N mice were analyzed for endogenous αSyn expression. **a)** Left and middle panels show detection of αSyn using the D37A6 antibody that specifically recognizes rodent αSyn in urinary bladder of WT mice at 60x and 20x magnifications, respectively. Right panel shows a 20x overview of the urinary bladder with DAPI, αSyn and peripherin expression. Scale bar is 50 µm. **b)** Detection of αSyn in transgenic human αSyn mice is negative (hu-aSyn mice are knock out for rodent aSyn) using the D37A6 antibody, indicating that αSyn expression is specific for mouse αSyn in urinary bladder. **c)** Expression of αSyn was detected in βIII tubulin-positive neurons of the detrusor muscle. Scale bar is 15 µm.

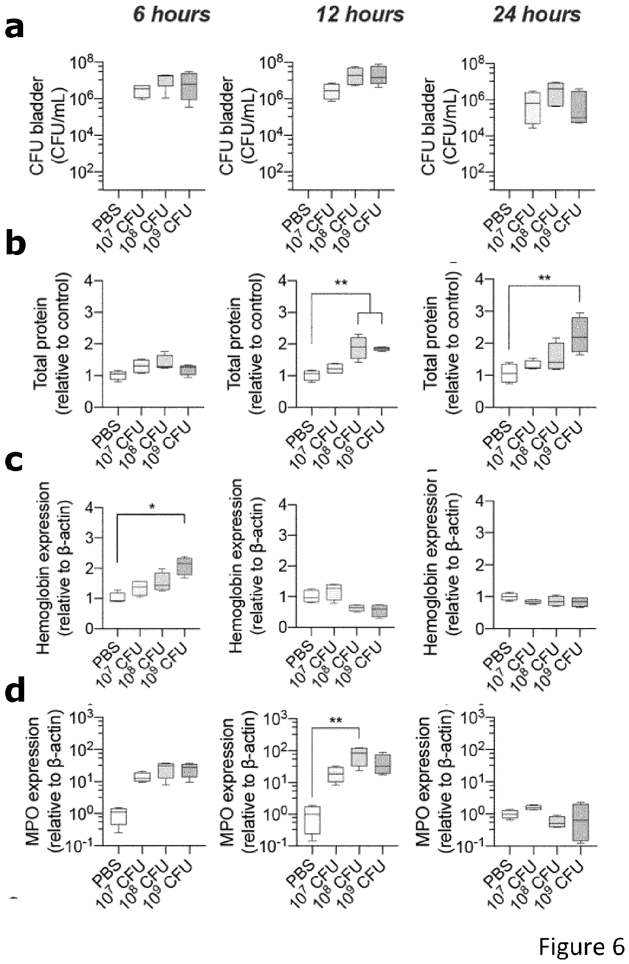

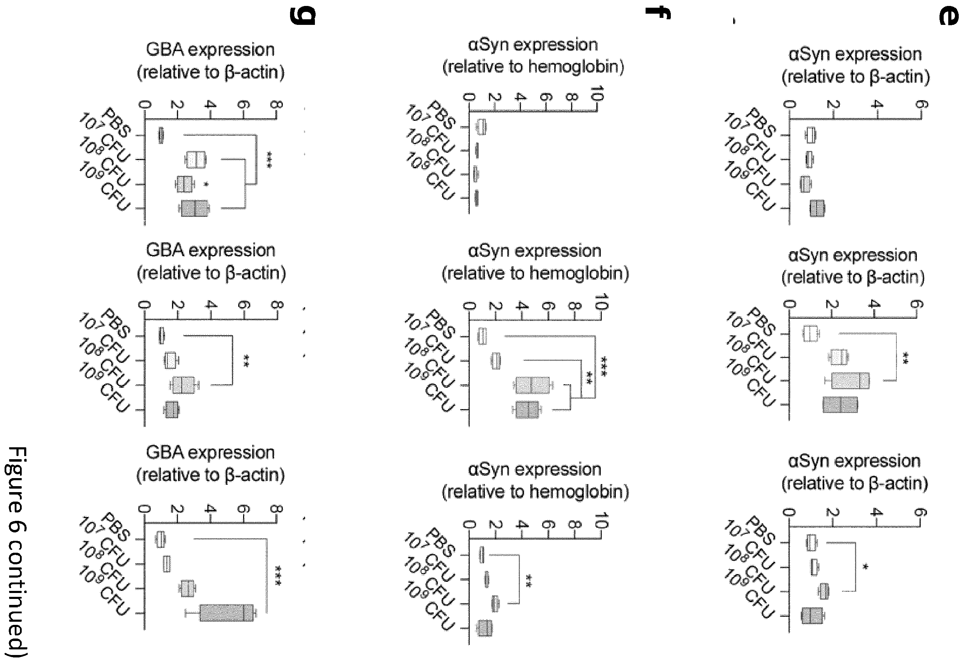

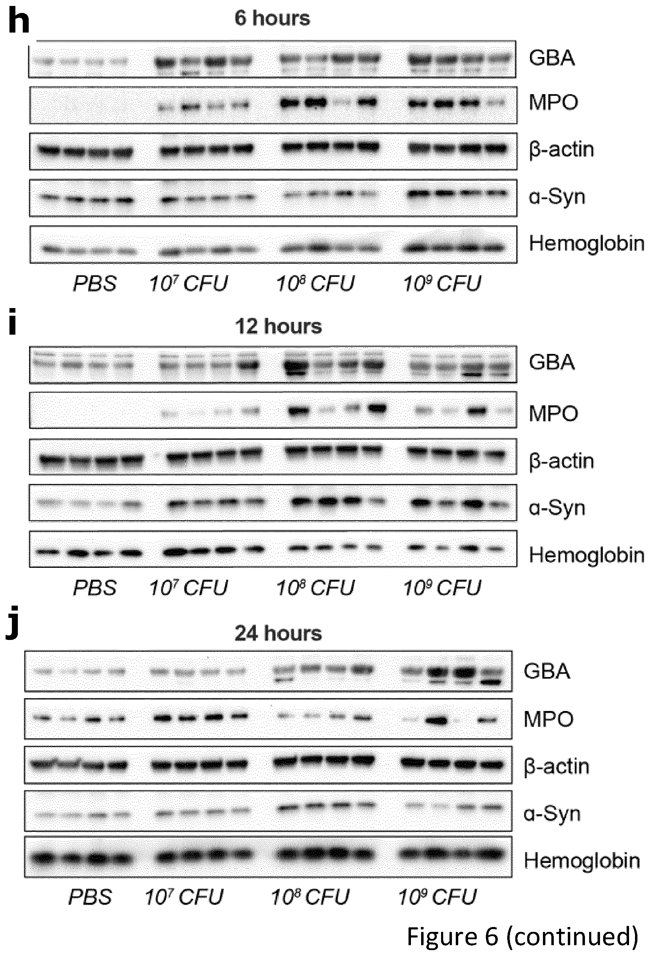

### Figure 6. αSyn expression varies with the strength of urinary tract infection in C57BL/6N mice.

WT C57BL/6N mice were acutely infected with varying doses of UPEC and analyzed at different time points for αSyn expression. **a)** To determine the relative αSyn response to different infectious triggers, animals were infected with 10⁷, 10⁸ or 10⁹ CFUs. Bladders isolated at 6, 12 and 24 hours showed similar infectious titers in urinary bladder of infected animals. **b)** Urinary bladders showed increased total protein content towards later time points suggestive of an active immune response. c) Relative levels of hemoglobin (versus β-actin) increase at the 6-hour time point in a dose-dependent way but remains unaltered during the later course of infection (n = 4, p^{∗} < 0.05 with one-way ANOVA and Bonferroni correction for multiple comparisons). **d)** MPO expression during different time points indicate neutrophil infiltration at earlier time points, with a peak at 12 hours of infection for 10⁸ CFU (n = 4, p^{∗∗} < 0.01 with Kruskal Wallis and Dunn's correction for multiple comparisons). **e)** Analysis of αSyn expression reveals increased levels at the 12- and 24-hour time points with the two higher injected doses of 10⁸ and 10⁹ CFU but not with 10⁷ CFU (n = 4, p^{∗∗} < 0.01 with one-way ANOVA and Bonferroni correction for multiple comparisons), **f)** which is significant after correcting for blood erythrocytes, an abundant source of αSyn (n = 4, p^{∗∗∗} < 0.001 with one-way ANOVA and Bonferroni correction for multiple comparisons). This indicates that the strength of the trigger determines αSyn expression levels. **g)** The lysosomal enzyme GBA, and PD risk factor, enriched in peripheral blood mononuclear cells and neutrophils is significantly elevated during the course of infection (n = 4, p^{∗∗∗} < 0.001 with one-way ANOVA and Bonferroni correction for multiple comparisons). **h-j)** Western Blot analysis of GBA indicates different glycosylated forms of the protein, with the lower bands suggesting removal of glycosylation and a shift to a lysosomal active form of the enzyme. Western blots from graphs at different time points and different doses **a-g)** are shown in **h-j).**

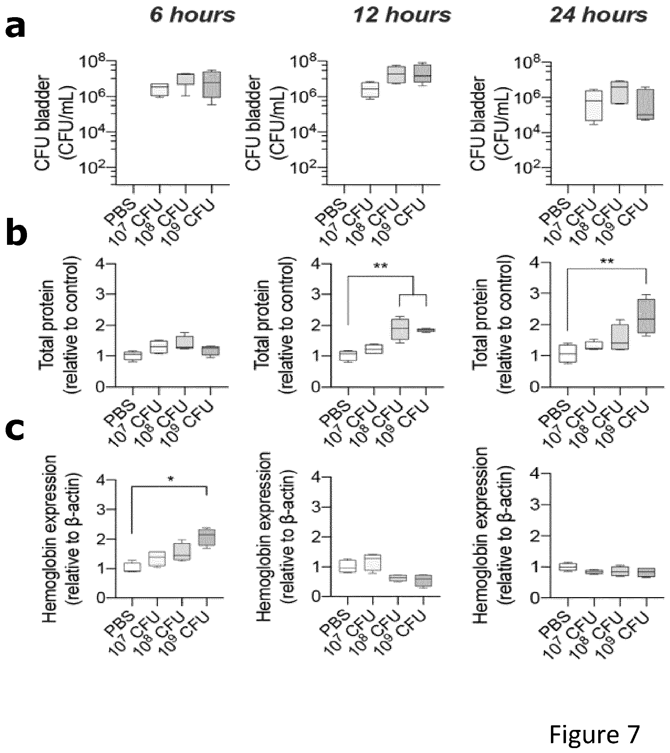

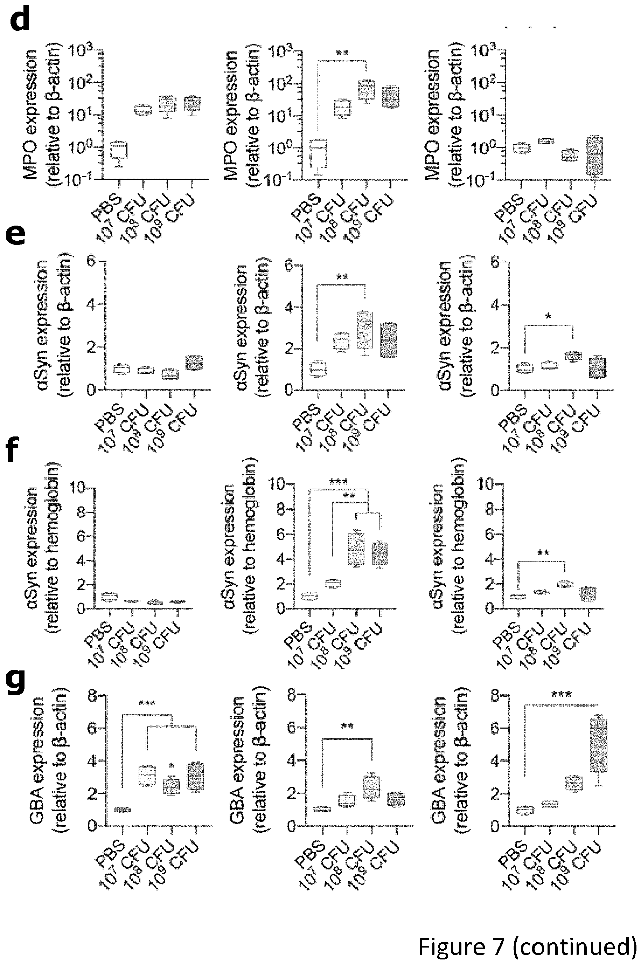

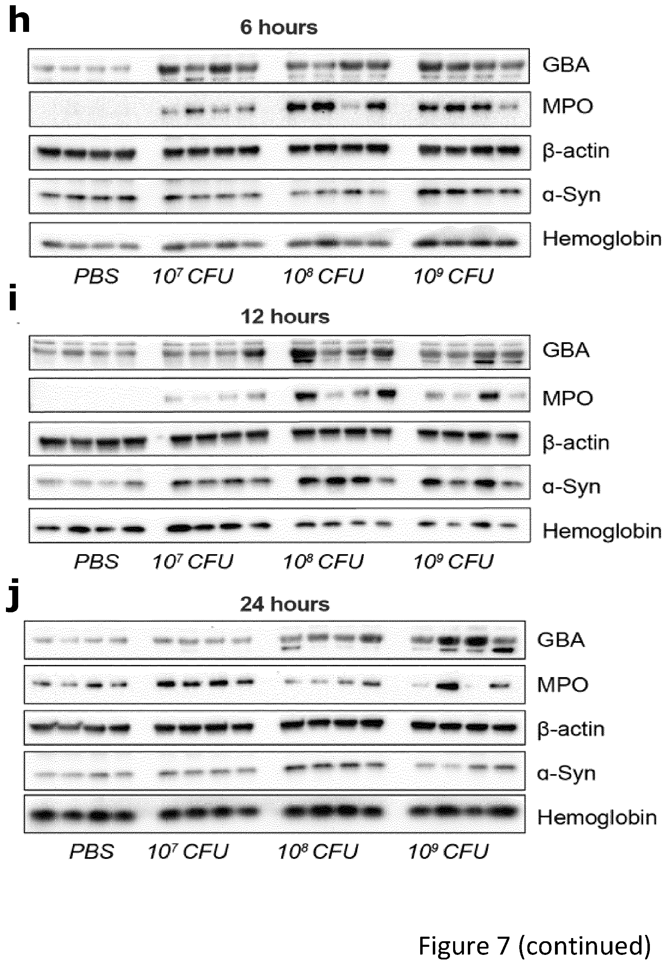

### Figure 7. αSyn expression varies with timing and the strength of urinary tract infection in C57BL/6J mice.

Small differences in the genetic background of mouse substrains can influence the immune phenotype. To examine how the genetic background could affected the observed response in C57BL/6N we examined the effects on αSyn during infection in C57BL/6J mice. WT C57BL/6J mice were acutely infected with varying doses of UPEC and analyzed at different time points for αSyn expression. a) Animals were infected with 107, 108 or 109 CFUs at different time points. Bladders isolated at 6, 12 and 24 hours showed decreasing infectious titers towards later time points (n = 4, p^{∗} < 0.05 with one-way ANOVA and Bonferroni correction for multiple comparisons). b) Urinary bladders showed increased total protein content for 109 CFU. c) Relative levels of hemoglobin (versus β-actin) are unaltered during the later course of infection. d) MPO expression at 6 hours indicate neutrophil infiltration for 109 CFU (n = 4, p^{∗} < 0.01 with Kruskal Wallis and Dunn's correction for multiple comparisons). e) Analysis of αSyn expression reveals increased levels at the 12-hour time point with 107 and 108 CFU (n = 4, p^{∗∗} < 0.01 with one-way ANOVA and Bonferroni correction for multiple comparisons), but not at 6 or 24 hours, f) which is significant after correcting for blood erythrocytes (n = 4, p^{∗} < 0.05 with one-way ANOVA and Bonferroni correction for multiple comparisons). g) GBA is significantly elevated after 6- and 12-hours of infection (n = 4, p^{∗∗∗} < 0.001 with one-way ANOVA and Bonferroni correction for multiple comparisons). Western blots from graphs at different time points and different doses a-g) are shown in h-j).

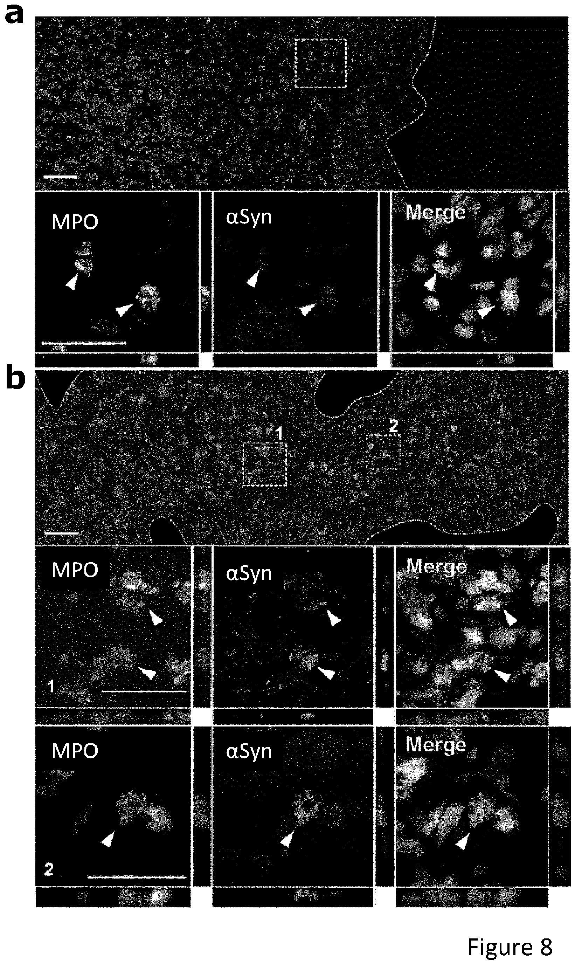

### Figure 8. Detection of αSyn in neutrophils of human urinary bladder during cystitis.

**a)** αSyn expression is low in resting neutrophils in the lamina propria of normal urinary bladder. Inset shows MPO-positive neutrophils and low levels of αSyn. Scale bar is 40 µm. **b)** A case of cystitis with edematous urothelium and widespread infiltration of neutrophils. Inset 1 shows αSyn deposited with MPO and decondensation of DAPI. Inset 2 shows an intact neutrophil with overlap between αSyn and MPO. Scale bar is 40 µm.

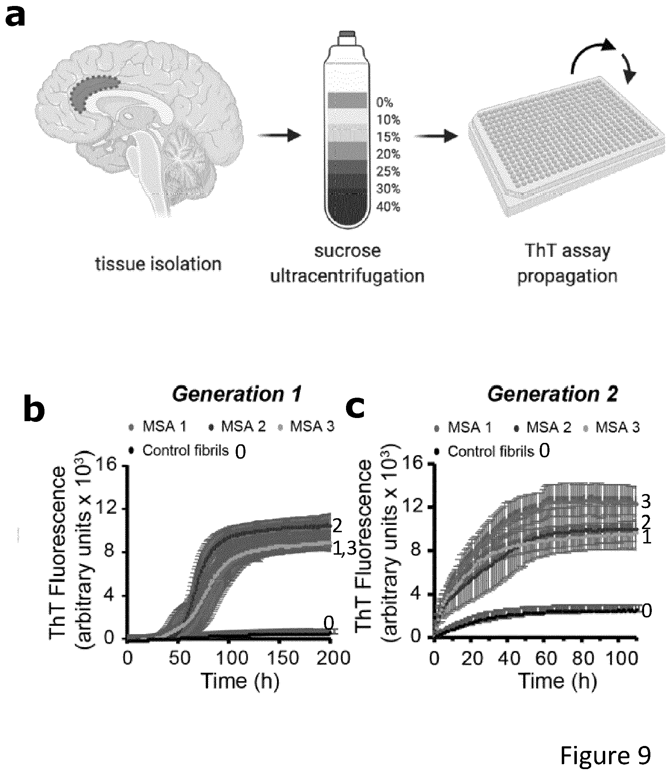

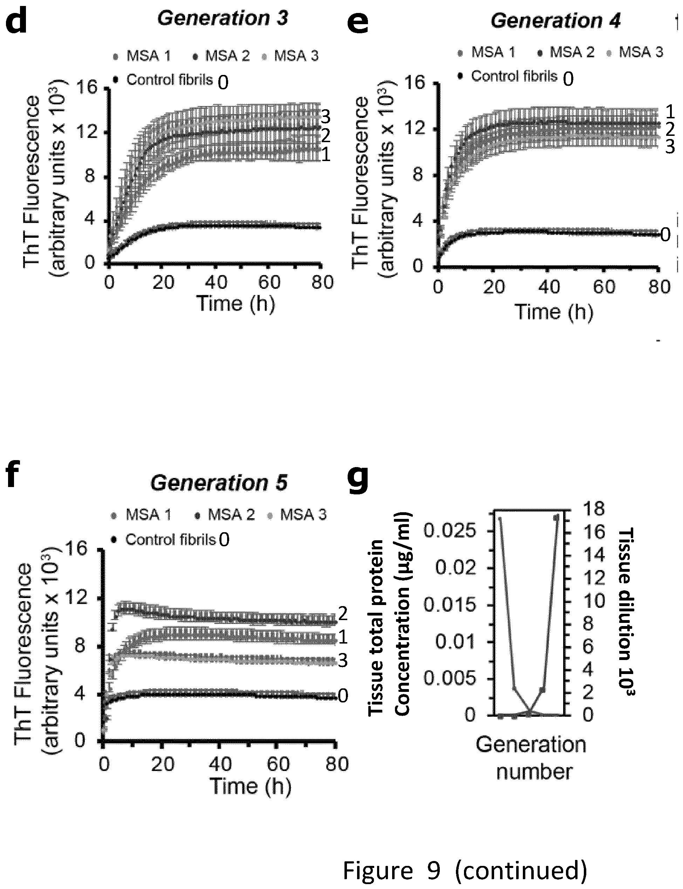

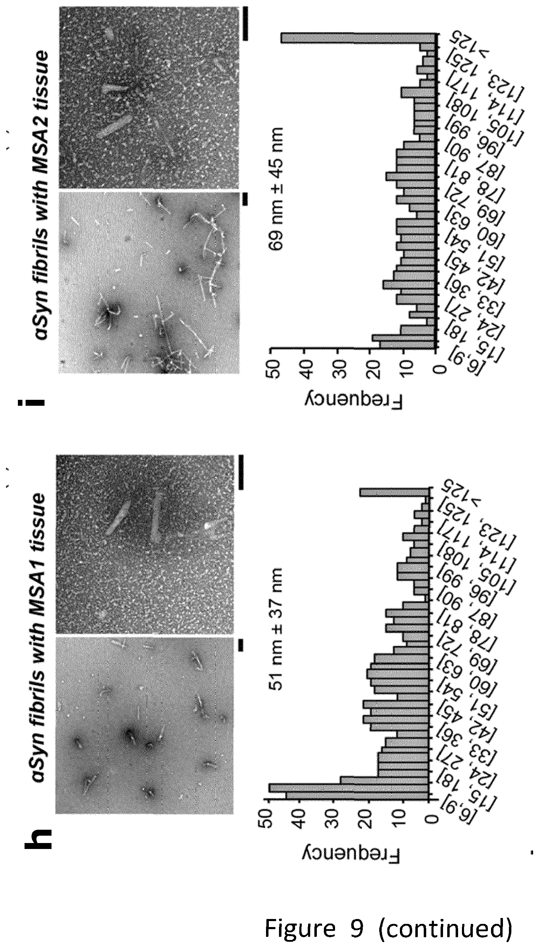

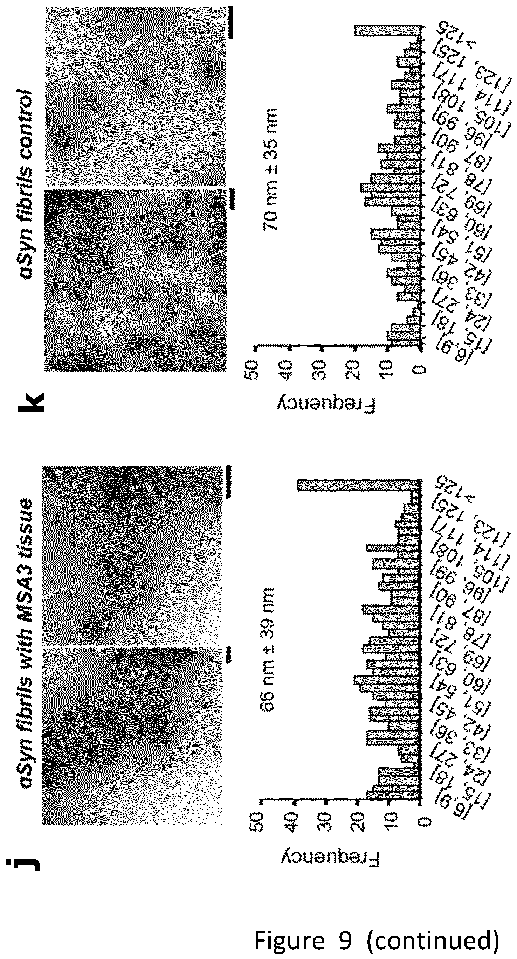

### Figure 9. Amplification of fibrils from brain tissues of patients with MSA.

**a)** Anterior cingulate tissues of three individuals with MSA were used to generate αSyn fibrils for the mouse-injection studies. Homogenized brain tissue was fractionated via sucrose ultracentrifugation and amplified using 384-well plates. Four to six wells were supplemented with 10 µM ThioflavinT (ThT) to monitor the progress of fibril formation. **b)** Fibril formation of recombinant αSyn was seeded with 10%-sucrose-MSA fractions. Control samples are fibrils formed by incubation of αSyn only.

Fibrils were propagated in five sequential reactions **(b-f),** and each fibril generation was formed by seeding αSyn polymerization with the fibrils collected from the previous reaction. MSA1, MSA2, and MSA3 - 10%-sucrose fractions of anterior cingulate tissues of three patients diagnosed with MSA (means = 4-6 technical replicates, SE). **g)** Total protein concentration of MSA-tissue seeds (left) and times dilution (right) are plotted for each generation of fibrils. Transmission electron microscopy images of **h)** MSA patient 1-, **i)** MSA patient 2-, **j)** MSA patient 3-, tissue-seeded and **k)** αSyn -only fibrils. Average length of sonicated fibrils measured in the electron micrographs are indicated for each patient. Animals were injected with samples in which three fibril batches generated with tissues of three MSA patients were combined in equimolar ratios. Scale bar is 100 nm.

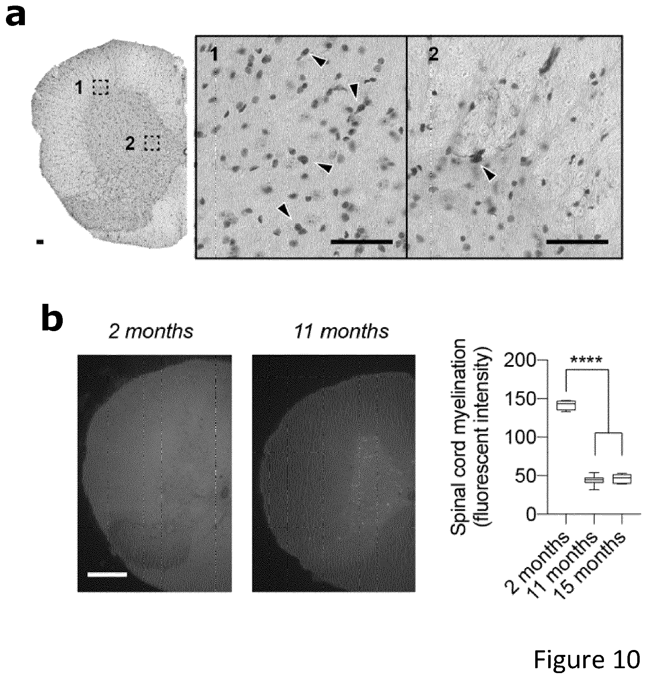

### Figure 10. MSA-like pathology in aged human αSyn transgenic mice.

a) At 11 months of age, PSer129-αSyn glial cytoplasmic inclusions (GCI) are detectable in transgenic human αSyn mice. Insets 1 and 2 show GCIs in the sacral spinal cord (dark arrows, scale bar is 100 µm). b) Detection of spinal cord myelin with Fluoromyelin shows demyelination at 11 and 15 months of age (n = 4, ^{∗∗∗∗}p<0.0001 with s.e.m. and one-way ANOVA with Bonferroni correction for multiple comparisons).

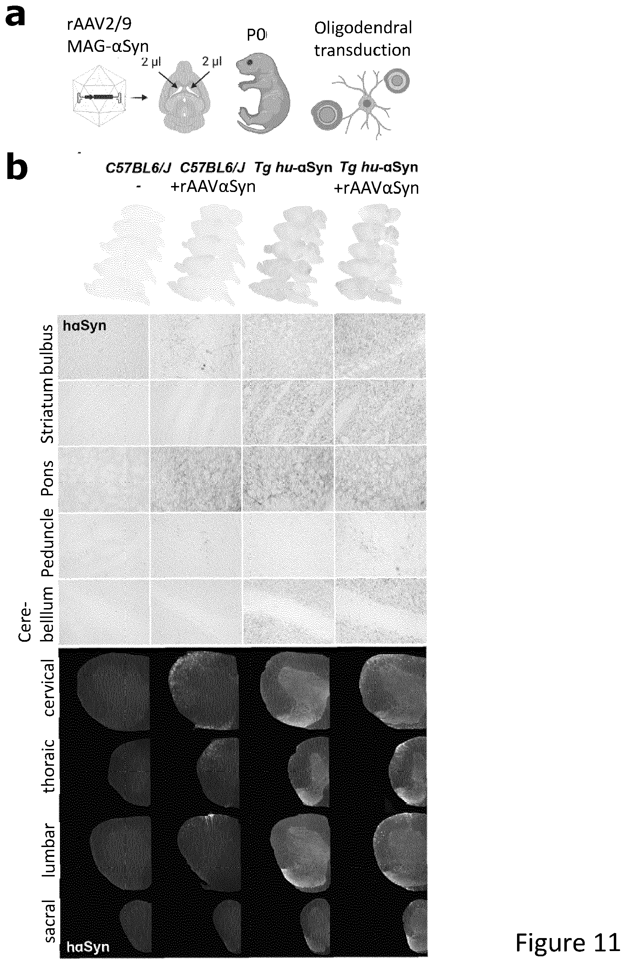

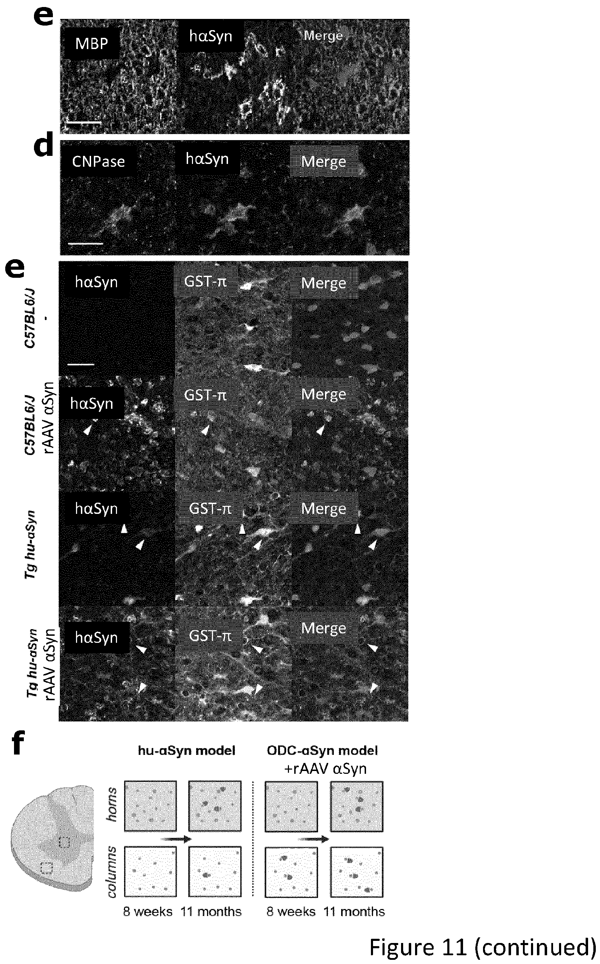

### Figure 11. Animal models used to study MSA disease progression in vivo.

**a)** Mouse neonates (P0) were bilaterally injected in the lateral ventricles with the oligodendrocyte expressing viral vector rAAV2/9-MAG αSyn. The vector expresses in oligodendrocytes with a specificity of over 95%. **b)** After intracebroventricular (ICV) delivery in C57BL/6J mice, there is detection of human αSyn with the human αSyn-specific antibody MJFR-1 in bulbus olfactorius, pons neuropil and the cerebellar peduncle of the cerebrum 21 days after injection. The expression of human αSyn from transgenic human αSyn mice (Tg (SNCA^{WT}) and the ICV delivered rAAV2/9 MAG-αSyn in mouse cerebrum cannot be distinguished. Closer examination of the spinal cord of C57BL/6J and transgenic human αSyn mice reveals that ICV delivery of the viral vector results in widespread αSyn expression in the anterior columns of the spinal cord. Confocal analysis of the mature oligodendroglial markers **c)** MBP and **d)** CNPase with and human αSyn shows that the expression of human αSyn is restricted to mature spinal cord oligodendrocytes (Scale bar indicates 40 µm). **e)** Oligodendroglial expression of human αSyn is absent in spinal cord of WT C57BL/6J mice but stain positive for C57BL/6J mice transduced with rAAV2/9 MAG-aSyn as indicated by the oligodendroglial marker GST-n (scale bar indicates 50 µm). Transgenic human αSyn mice express detectable levels of oligodendroglial αSyn under basal conditions and ICV injection with rAAV2/9 MAG-aSyn results in more widespread expression of human αSyn in spinal cord oligodendroglial cell bodies and myelin tracts of the anterior columns **f)** Summary of the two models used to study disease progression in MSA. Transgenic human αSyn mice (the hu-aSyn model) and transgenic human αSyn mice injected with rAAV2/9 MAG-aSyn (the ODC-model) differentially express oligodendroglial human αSyn. The hu-aSyn model expresses relatively low levels of oligodendroglial human αSyn at 8 weeks and progressively develops MSA-like features. The ODC-aSyn model expresses αSyn in anterior column at 8 weeks and progressively develops additional pathology including myelin loss and spinal GCIs.

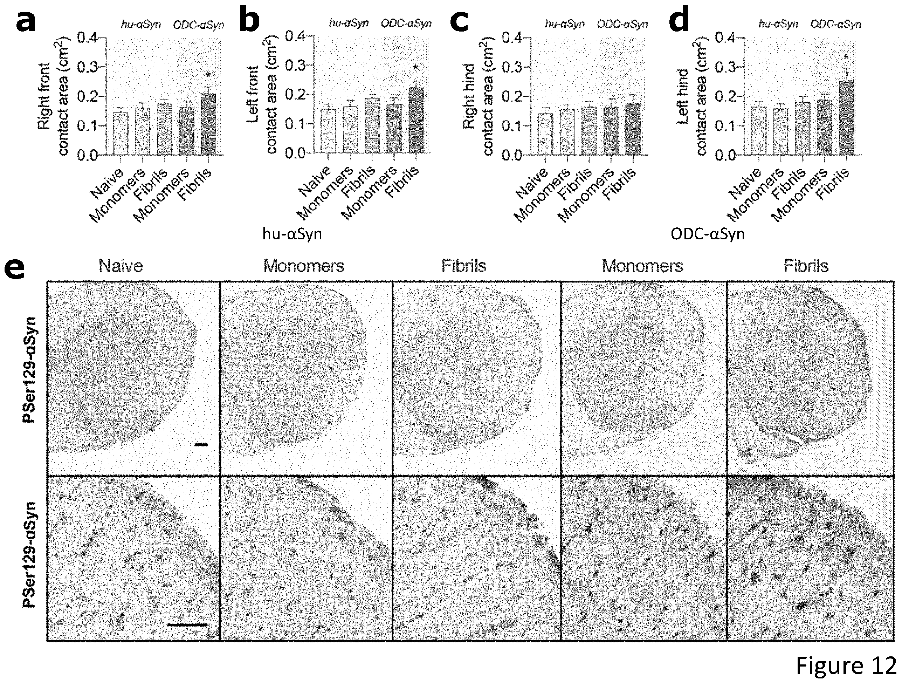

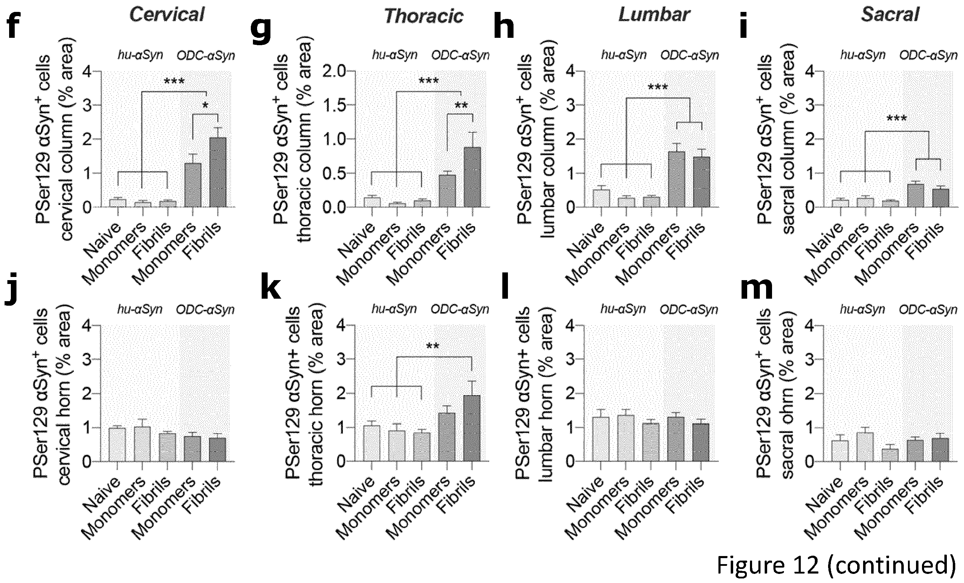

### Figure 12. Characterization of behavioral deficits and pathological markers after injection of MSA fibrils in mouse urinary bladder.

Animals were injected with MSA fibrils or αSyn monomers at 8 weeks of age and followed up for behavioral deficits. At 9 months after injection of MSA fibrils or monomers, fine motor control deficits in **a)** right and **b)** left forepaws and **c)** right and **d)** left hindpaws are apparent in ODC-aSyn mice injected with MSA fibrils but not after injection with monomers (n ≥ 6, s.e.m. ^{∗}p<0.05 with mixed-effects analysis of two-way ANOVA and Tukey post hoc correction for multiple comparison). No motor deficits are observed when injecting recombinant protein in hu-aSyn mice. **e)** Analysis of PSer129-αSyn inclusions in mouse spinal cord (scale bar 100 µm) quantified in **f-m)** shows increased synucleinopathy in spinal cord white matter columns of the **f)** cervical and **g)** thoracic segments and the thoracic anterior horn compared to ODC-aSyn injected with monomers or control animals (n ≥ 6, s.e.m. ^{∗}p<0.05 with mixed-effects analysis of two-way ANOVA and Tukey post hoc correction for multiple comparison). The increase in PSer129-αSyn immunoreactivity in ODC-aSyn animals compared to hu-aSyn mice injected with monomers could be due to viral vector mediated expression of human αSyn in oligodendrocytes. No additional pathology is observed in hu-aSyn mice injected with monomers of MSA fibrils.

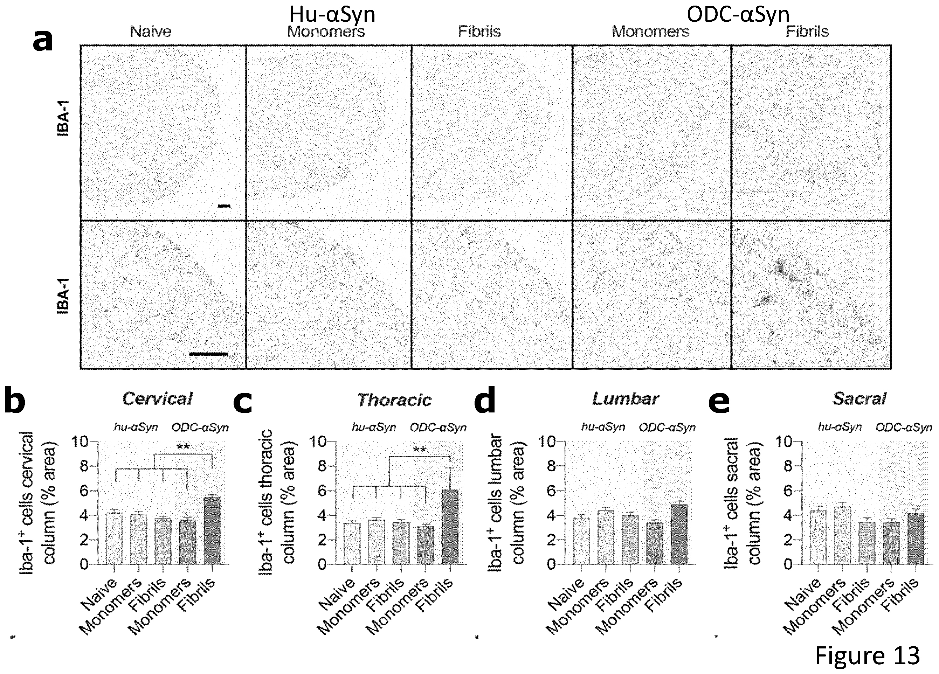

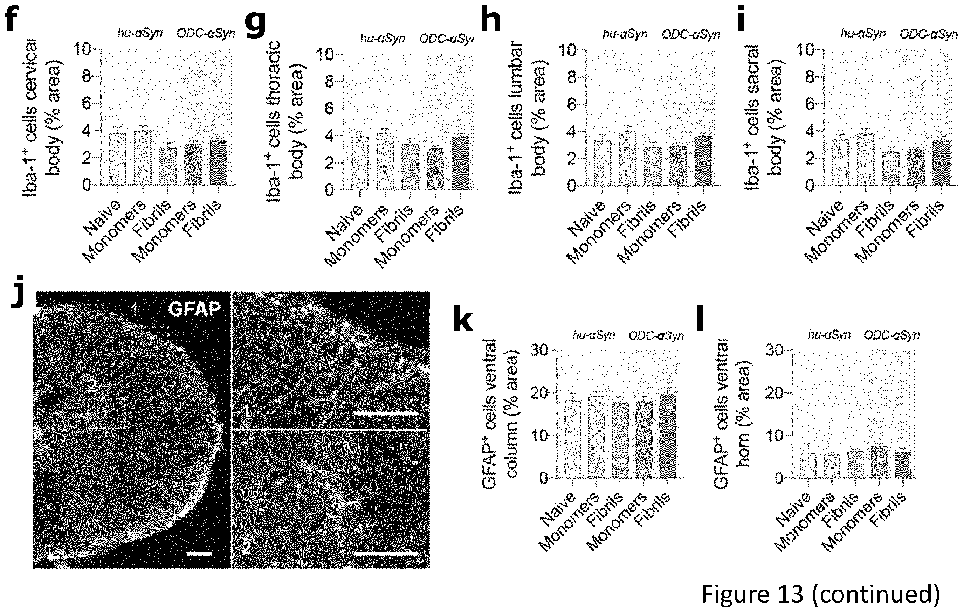

### Figure 13. Characterization of inflammatory markers in spinal cord after injection of MSA fibrils in mouse urinary bladder.

**a)** Immunohistochemical analysis of Iba-1 in spinal cord (scale bar 100 µm) quantified in **b-i)** shows microglial activation in the spinal cord white matter anterior columns of cervival and thoracic segments of ODC-aSyn animals injected with MSA fibrils (n ≥ 6, s.e.m. ^{∗}p<0.05 with mixed-effects analysis of two-way ANOVA and Tukey post hoc correction for multiple comparison). No microglial activation was observed ODC-aSyn animals injected with monomers or in any other experimental conditions. **j)** Analysis of GFAP in spinal cord (200 µm scale bar) shows no differences in astrocyte response in the **k)** thoracic white matter or **I)** thoracic anterior horn between different experimental conditions.

The etiopathology of MSA is largely unknown, and it has been suggested to be due to a combination of environmental triggers and low polygenic risk. Genome wide association studies have suffered from being relatively small and as a consequence have largely been inconclusive without any clearly significant risk loci emerging across studies. The search for causative environmental factors has also not yielded evidence for how MSA starts, also possibly due to the disease being very heterogeneous. However, clues from the neuropathology of different forms of MSA, as well as clinical characteristics of the earliest stages of the disease might be instructive. Thus, characteristic neuropathology of MSA includes αSyn aggregates in oligodendrocytes and involvement of spinal cord and lower brain stem and patients frequently report numerous UTIs during the disease prodrome, before the onset of motor symptoms. After development of motor signs and formal diagnosis, UTIs remain common, possibly in part due to impaired bladder emptying. We propose that a peripheral bacterial infection can act as a trigger of synucleinopathy in the MSA disease process, and here we present supporting evidence from human tissues, cell culture and mouse models.

When we examined urinary bladders of MSA patients and age-matched controls, we found numerous αSyn deposits consisting post-translationally modified (phosphorylated at serine residue 129, widely viewed as one likely sign of pathological aggregation) protein. These αSyn deposits were present throughout the tissue, most densely around blood vessels. These αSyn deposits show that αSyn can accumulate in a peripheral organ that is affected early in MSA. The dense distribution of αSyn around blood vessels raises the possibility that the *de novo* aggregation of αSyn is not seeded by neuronal aSyn, but that the source might be from another cell type. Given the high occurrence of UTIs in the general population [Flores-Mireles et al. Nat. Publ. Gr. 13, 269-284 (2015)], the high frequency of MSA patients developing chronic UTIs and the role of αSyn the immune system [Harms et al. Acta Neuropathol. 141, 527-545 (2021)], we investigated if αSyn expression and aggregation could be triggered by a UTI. We used a well characterized mouse model of UTI to study the αSyn response to bacterial infection in WT mice and found that αSyn expression significantly increases during the early steps of acute infection with the formation of insoluble αSyn aggregates. This response was transient and expression of αSyn was found in sites with edema of the lamina propria, concurrent with infiltration of activated innate immune cells. The increase of αSyn was dependent on the number of infecting UPEC, with higher numbers of bacteria leading to a greater increase in αSyn expression. Notably, upon histopathological examination of the urinary bladder walls, adjacent to blood vessels of the lamina propria we observed neutrophils surrounded by morphological features indicative of extracellular traps (NETs). Strikingly, these NETs were also consistently immunoreactive for αSyn.

In parallel to the observations in infected mouse urinary bladders *in vivo,* we found that αSyn is expressed in primary human neutrophils and in neutrophils of the human urinary bladder. *In vitro* stimulation of human neutrophils with UPEC caused the release of αSyn and also in biopsies of cases with acute interstitial cystitis, we detected aSyn-immunoreactivity in neutrophils intra- and extracellularly. We focused our attention on the expression of αSyn in neutrophils, but other immune cells such other types of granulocytes, macrophages and lymphocytes, are also known to express αSyn [Grozdanov & Danzer Front. Cell Dev. Biol. 8, 1-6 (2020)]. It is likely that these immune cells further contribute to changes in αSyn levels in the wall of the infected urinary bladder, and they deserve further investigation in the future, but these other immune cell types are not known to release their intracellular content like neutrophils. What might be the functional role of αSyn in the context of innate immunity? It has been shown that αSyn assists in innate immune cell function via its role in membrane binding and membrane modelling [Gardai et al. PLoS One 8, e71634--21 (2013)]. αSyn favorably binds with membranes of high curvature such as mitochondria, small synaptic vesicles, phagophores or granules *(9)*. Increasing levels of αSyn in phagocytic cells results in increased membrane binding with inhibitory effects on vesicle dynamics, inhibition of phagocytosis and reduced uptake of bacteria [Gardai et al. cited above]. During an infectious trigger, key bactericidal enzymes generate tyrosine and nitrogen radicals that are released to crosslink NET proteins [Papayannopoulos Nat. Rev. Immunol. 18, 134-147 (2017)]. These highly reactive and oxidative conditions are known to be conducive to αSyn aggregation and since we detected aggregated αSyn in whole bladder homogenates, we propose that radicals released by neutrophils might be responsible for catalyzing the *de novo* assembly of αSyn into high molecular weight assemblies.

Because of the presence of αSyn within NETs, we further investigated the role of αSyn during NETosis. Via live imaging of fluorescently labelled αSyn we observed that with increasing levels of aSyn, dHL60 cells more efficiently releases NETs. In line with our previous observations in mouse and human urinary bladder, we found αSyn also within these extracellular NETs. MPO-mediated activation of neutrophil elastase is important for the degradation of actin and the inhibition of phagocytosis in the steps before NET formation. Phagocytic inhibition of phagophores allows neutrophil elastase to translocate to the nucleus and cause nuclear decondensation by histones, thereby releasing NETs [Kolaczkowska & Kubes, cited above; Papayannopoulos, cited above]. As a known regulator of endocytosis and phagocytosis, αSyn is well positioned within this pathway to prevent sequestration of enzymes into phagosomes and facilitate NET formation. In our *in vitro* assay, it was sufficient to increase the levels of αSyn to promote NET formation.

We and others have suggested that αSyn aggregation might be a common phenomenon in peripheral tissues, but only when the host is susceptible (e.g. due to genetic predisposition or aging) will the aggregated αSyn propagate pathology from the periphery to the CNS. Consistent with this idea, we detected αSyn deposits in urinary bladder not only of people with MSA but also in control subjects with no clinical history of neurodegenerative disease. Early appearance of urinary and erectile dysfunction occurs in some MSA patients and it has been proposed that they might represent a specific subtype of MSA. Classically, patients develop urinary retention with the appearance of neurological signs. However, a subset of MSA patients experience urogenital symptoms as their first symptom. These patients typically exhibit urinary urgency and incontinence, after which they then develop urinary retention or other voiding difficulties. During the early stages of MSA, erectile dysfunction, anorgasmia and gut symptoms become more apparent suggesting involvement of the lower spinal cord or infrasacral regions. These prodromal MSA features worsen over time, preceding the development of motor symptoms several years later leading to clinical diagnosis of probable MSA. Although not fully understood, the pathophysiological basis for urogenital dysfunction in MSA has been suggested to be due to neurodegeneration in the basal ganglia, the pons, the cerebellum, anterior horn neurons in Onuf's nucleus, the intermediolateral cell columns and the white matter spinal tracts that convey the sympathetic and parasympathetic fibers of the urinary bladder to the brain [Fowler et al. Nat. Rev. Neurosci. 9, 453-466 (2008)].

Several studies have shown that peripheral injection of fibrillar αSyn can trigger neuropathology in wild type and transgenic animals via seeding the aggregation of endogenous αSyn protein [Challis et al. Nat. Neurosci., 1-27 (2020); Kim et al. Neuron 103, 627--641.e7 (2019)]. However, prior studies have not demonstrated oligodendroglial pathology that resemble that seen in MSA. To test if aggregated αSyn can spread from the urogenital tract to the CNS and seed aggregation leading to functional deficits, we injected αSyn fibrils, amplified from human MSA brain, into the urinary bladder of hu-aSyn and OCD αSyn mice. Hu-aSyn mice express normal levels of αSyn in neurons of the CNS and have low, barely detectable, levels of αSyn in oligodendrocytes of the spinal cord. By contrast, OCD-aSyn mice express additional hu-aSyn in spinal cord oligodendrocytes of the white matter tracts. Nine months after injection of amplified human MSA fibrils into the urinary bladder of OCD-aSyn mice, we observed that injection of αSyn MSA fibrils into the wall of the urinary bladder led to pathological changes analogous to those seen in MSA in the spinal cord. Furthermore, the mice developed changes in urinary bladder function and gait abnormalities, once again in analogy to what is seen in MSA. These changes were absent in the hu-aSyn mice and injection of equimolar non-aggregated, monomeric assemblies of αSyn in the urinary bladder did also not lead to pathology in any of the paradigms tested. The reason for the absence of pathology in hu-aSyn mice could be multifold, but it suggests that in this paradigm the host environment is not susceptible for the propagation of αSyn or alternatively, that we did not wait long enough to allow the pathology to develop after urinary bladder injection of MSA fibrils.

In order for disease-specific fibrils of MSA to develop *in vivo,* αSyn seeds have to amplify with monomeric αSyn in oligodendrocytes. Therefore, monomeric αSyn has to be expressed in oligodendrocytes since the seeding and amplification of MSA strains cannot occur otherwise. Under normal conditions, expression of αSyn in oligodendrocytes is low, but recent reports have shown that oligodendroglial expression of αSyn can increase in response to inflammatory insults or during active demyelination in the spinal cord. The OCD-aSyn mice mimic such an environment and they are more permissive to the development of MSA, by specifically overexpressing monomeric human αSyn in oligodendrocytes.

In this study we provide support for the idea that a bacterial infection in a peripheral organ can under certain circumstances act as a trigger of synucleinopathy, with neutrophil leucocytes playing a role. We also present a mouse model in which, under permissive conditions with αSyn overexpression in oligodendrocytes, the injection of αSyn fibrils amplified from MSA brain into the urinary bladder can generate spinal cord pathology with some cellular and anatomical features in common with MSA, as well as behavioral deficits. These findings, coupled with numerous reports of urogenital dysfunction in the MSA prodrome, suggest that in some cases of MSA the disease process might start in the urogenital region. Future studies should identify if early changes in the urogenital organs can act as therapeutic targets in MSA.

### EXAMPLES

### Example 1. Detection of pathological αSyn in human urinary bladder

αSyn is expressed in synapses of peripheral neurons, but immunohistochemical detection of endogenous, non-aggregated, αSyn in human urinary bladder has not been described previously. The human urinary bladder is densely innervated by sensory and somatic fibers in the suburothelial and urothelial layers [Fowler et al. Nat. Rev. Neurosci. 9, 453-466 (2008)]. To detect endogenous αSyn in human urinary bladder, HIER-mediated antigen retrieval was performed on paraffin-embedded urinary bladder tissue after which immunohistochemical staining was done using the human-specific αSyn MFJR-1 antibody that recognizes different assembly states of αSyn. Using this method, we detect widespread and punctuate αSyn expression within intramural ganglia (Fig. 1a) and neuronal synapses of the human urinary bladder (Fig. 1b). αSyn is expressed throughout the detrusor muscle but also the lamina propria in very close proximity of the bladder lumen (Fig. 5a). Control and MSA subjects have similar expression levels of αSyn as monitored using fluorescent detection with the MJFR-1 antibody (Fig. 1c).

We confirm the presence of αSyn in human urinary bladder and recently it was shown that in MSA urinary bladder tissue, αSyn can form aggregated PSer129-αSyn-immunoreactive inclusions. To further expand on these findings, we assessed urinary bladder tissue from 8 MSA patients and 8 age- and gender-matched controls with an average age of approximately 70 years old (Table 1) for PSer129-αSyn and aggregated αSyn (FILA-1).

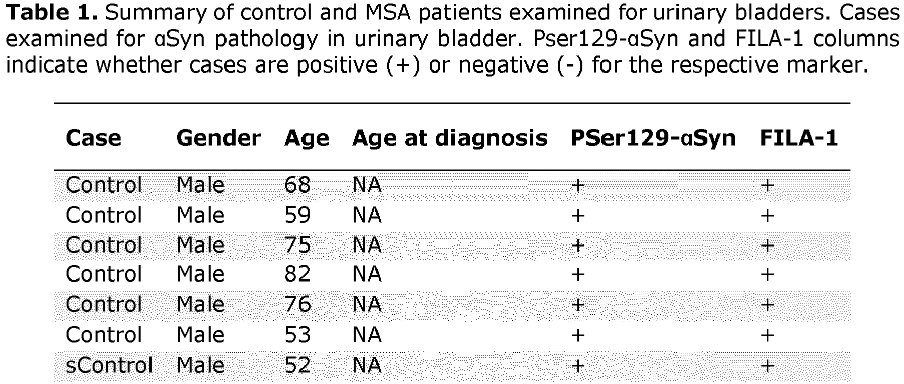

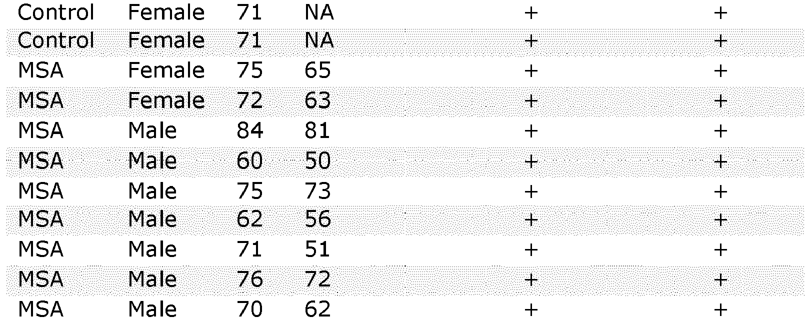

Interestingly, when examining the presence of pathological αSyn we find positive immunohistochemical staining for different pathological markers of αSyn in both control and MSA cases (Fig. 1d-e). PSer129-αSyn and FILA-1 antibody detection of pathological αSyn is apparent as a punctuate pattern throughout bladder within the detrusor muscle and lamina propria (Fig. 1d). In addition, we find dense accumulation of PSer129-αSyn and aggregated αSyn around blood vessels (Fig. 1e). Together, this shows that αSyn is expressed throughout the urinary bladder and that in the cases examined here, PSer129-αSyn-immunoreactive (potentially pathological) αSyn is present in the urinary bladder of both control subjects and MSA patients. This suggests that peripheral aggregates of αSyn in the urinary bladder are prevalent and which raises the possibility that aggregation of αSyn could be triggered by different environmental factors, such as bacterial or viral infections. In a permissive environment, aggregated αSyn is thereby potentially set to propagate from the peripheral organ to the CNS via the innervating nerves and cause pathology by seeding further aggregation in cells in the CNS.

### Example 2. Urinary tract infections trigger aggregation of endogenous αSyn

Because of the presence of pathological αSyn in human urinary bladder, and given the putative role of αSyn in the immune response, we decided to investigate whether αSyn aggregation could be triggered peripherally by an immune-related mechanism. Since MSA patients frequently develop UTI, even during the MSA prodrome, we chose to use a well-characterized animal model of UTI *(28)* to study the involvement of αSyn in the immune response during infection [Fowler et al. Nat. Rev. Neurosci. 9, 453-466 (2008)].

By infecting WT C57BL/6N mice with 10⁸ CFU of UPEC we assessed endogenous αSyn expression during acute infection (Fig. 2a, b). After 12 hours of infection, we observe a greater than threefold increase in expression of αSyn in urinary bladder via Western blot analysis of whole urinary bladder homogenates (Fig. 2c, d). This increase is also significant after correcting for potential contamination of αSyn from blood erythrocytes, a potential source of αSyn which could increase in abundance during hemolysis or hemorrhagic inflammation in UTI (Fig. 5e, f). At longer time points, the expression of αSyn is reduced and in contrast to infection with high titers of UPEC (10⁸ CFU and 10⁹ CFU), infection with 10⁷ CFU yields no significant increase of αSyn at 6, 12 or 24 hours post infection (Fig. 5e, f). This shows that the increase and kinetics of αSyn expression depend on the timing and the strength of the infectious trigger.

To visualize the expression of αSyn in mouse urinary bladder during infection, we used the rodent-specific αSyn D37A6 antibody, that specifically binds endogenous αSyn to perform immunohistochemical detection (Fig. 5a, b). Under normal conditions, endogenous αSyn is expressed in mouse urinary bladder neurons, where it colocalizes with neuronal markers with a diffuse or punctuate distribution throughout the bladder mucosa and detrusor muscle (Fig. 5 a, c). During infection, αSyn expression coincides with the infiltration of neutrophil leucocytes (Fig. 2d), as indicated by increased levels of MPO protein expression. Confocal analysis of sections through the wall of infected urinary bladder shows that in addition to neuronal aSyn, polynuclear granulocytes positive for the neutrophil marker myeloperoxidase (MPO) express αSyn (Fig. 2f). Correspondingly, at 12 hours after infection, the increase of αSyn is significantly accentuated within the lamina propria, quantified in Fig. 2h. Coupled with the fact that we could not detect any observable change in αSyn or PSer129-αSyn expression in neurons, this suggests that the αSyn expression we monitored might originate from the innate immune response, specifically due to the invasion of neutrophil leucocytes.

To further investigate the effect of infection on αSyn expression or protein misfolding, we isolated infected urinary bladders to examine the assembly state of αSyn. During the earliest steps of infection, neutrophils have an important role as effectors of inflammation in the urinary tract during infection [Abraham & Miao Nat. Rev. Immunol. 15, 655-663 (2015)]. Neutrophils create an oxidative environment to kill bacteria or other types of pathogens [Kolaczkowska & Kubes Nat. Rev. Immunol. 159-175 (2013)] but oxidative conditions are also known to efficiently catalyze aggregation of αSyn [Uversky et al., Mol. Brain Res. 134, 84-102 (2005)]. Given the sharp increase of αSyn expression and the reactive environment created by infection we examined if infections could trigger αSyn aggregation *in vivo.* Infected urinary bladders (10⁸ CFU) were isolated at the 12-hour timepoint and homogenized under denaturing conditions with 1% sarkosyl. After treatment, we detect aggregated αSyn in animals treated with UPEC but not in PBS treated animals (Fig. 2b). This shows that the *de novo* aggregation of αSyn can be triggered in the urinary bladder in response to a bacterial infection.

Next to their microbicidal role, neutrophils have an important cooperative effector function on macrophages resulting in reciprocal microbicidal and phagocytic activities during infection [Silva J. Leukoc. Biol. 89, 675-683 (2011)]. Resolution of inflammation relies on the fast clearance of granulocytes by phagocytic cells. To test the presence of phagocytic blood mononuclear cells (PBMCs), we analyzed the expression of the lysosomal enzyme glucocerebrosidase (GBA). GBA can be used as a marker for lysosomes of both phagocytic granulocytes and PBMCs in which it is enriched [Liel et al. Blood 83, 2646-2653 (1994); Aflaki et al. Sci. Transl. Med. 6, 240ra73--240ra73 (2014)]. During early and later time points of acute infection, we observe increased expression of GBA, corresponding with granulocyte and PBMC activity (Fig. 6 h-j). This suggests that after the initial increase of aSyn, the expression of αSyn diminishes during resolution of the infection, possibly with the removal of neutrophils.

### Example 3. αSyn is released as part of neutrophil extracellular traps

As a unique microbicidal strategy during host defense, neutrophils can form neutrophil extracellular traps (NETs). NETs are web-like chromatin traps that allow neutrophils to trap pathogens while releasing their enzymatic and reactive intracellular contents [Kolaczkowska & Kubes cited above]. Because of the strong association of αSyn with small membrane organelles and granules we tested if infection could trigger the release of αSyn from neutrophils and therefore if it could be a source of αSyn accumulation in peripheral tissue. We examined UPEC infected mouse urinary bladders at 12 hours after infection for the expression of αSyn in NETs. NETs are formed by DNA, histones and granular proteins and colocalization of these different structures is indicative of NETs [Kolaczkowska & Kubes, cited above]. Upon examination of MPO and histones (H2B) with aSyn, we detect aSyn-positive NETs in urinary bladder in close vicinity to blood vessels of the lamina propria (Fig. 3a). The intensity profiles of the three markers indicate a strong overlap as well as the confocal images that show that αSyn localizes with MPO and H2B (Fig. 3b, c).

To further examine the presence of αSyn in neutrophils, we stimulated isolated human primary neutrophils with UPEC. Under non-stimulated conditions, αSyn is detected intracellularly in primary human neutrophils throughout the cytoplasm using the human-specific αSyn MFJR-1 antibody (Fig. 3d, upper panel). However, during stimulation with UPEC, αSyn is detected with the DNA that is extracellular (Fig. 3d, lower panel) indicating that αSyn is released from neutrophils exposed to bacteria *in vitro.* Next, we examined the expression of αSyn in neutrophils from biopsies of human urinary bladder. We examined the urinary bladder for neutrophil infiltration and αSyn expression in tissue from 25 different individuals, including those with cystitis and those with no known underlying condition in the urinary bladder. In normal, non-inflamed human urinary bladder we detected αSyn in neurons, but there was no aSyn-immunoreactivity in the sparse circulating neutrophils in the walls of the bladders (Fig. 3e, upper panel and Fig. 8a). However, in cases with interstitial cystitis with marked infiltration of neutrophils, we detect expression of αSyn in neutrophils in 4 out of 11 cases (Fig. 3e, lower panel and Table 2).

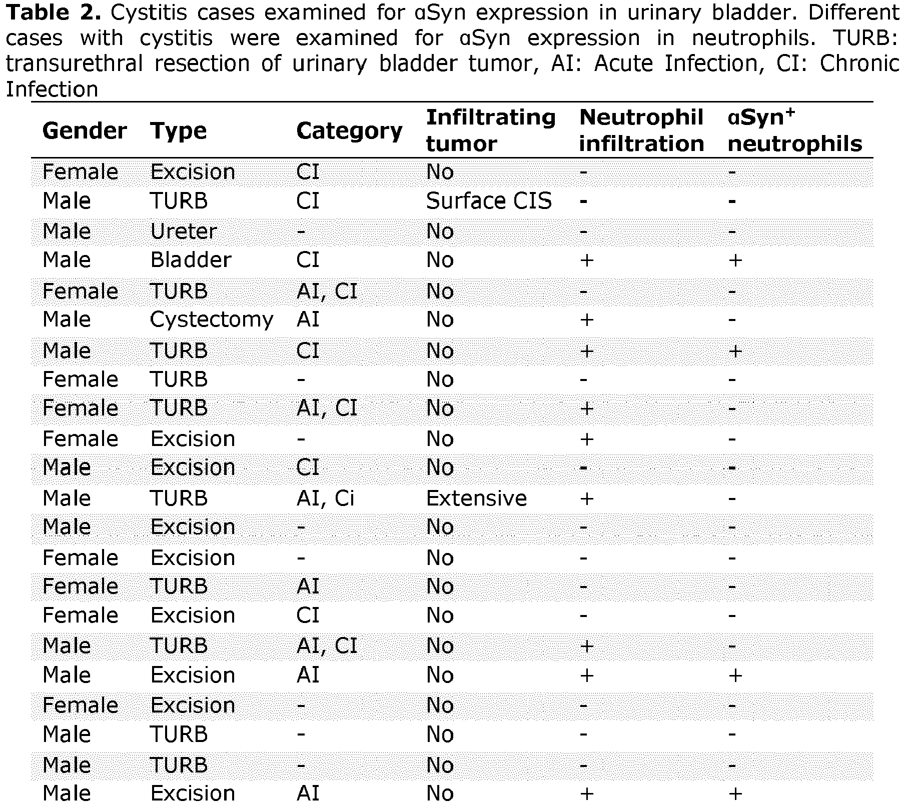

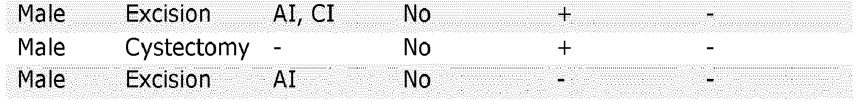

In cases with detectable levels of aSyn, the expression of αSyn was co-localized intra- and extracellularly with DNA or MPO. Neutrophils are known have a very short circulatory lifespan of a few, hours although they can be primed at the site of activation, which expands their longevity [Kolaczkowska & Kubes, cited above]. The short window in which αSyn is expressed during infection in our mouse model and the selective presence of αSyn in infiltrating neutrophils of human urinary bladder, suggests that αSyn is present in short-lived neutrophils that have invaded the wall of the infected bladder in inflammatory conditions.

The presence of αSyn in neutrophils suggest a possible role of αSyn in innate immune function. αSyn influences multiple steps of the endo- and exocytosis pathways and increasing levels of αSyn are known to inhibit phagocytosis in peripheral blood mononuclear cells during infection, via membrane binding and its inhibitory role on membrane fusion [Gardai et al. cited above]. Before NET formation, neutrophil elastase is activated by MPO via oxidation after which neutrophil elastase is released from azurophilic granules [Kolaczkowska & Kubes, cited above; Papayannopoulos cited above]. During these steps, neutrophil elastase inhibits the fusion of azurophilic granules with phagophores thereby preventing its sequestration away from the nucleus to initiate chromatin decondensation and NETosis [Kolaczkowska & Kubes, cited above; Papayannopoulos cited above]. Given the role of αSyn in vesicle dynamics, and its inhibitory effects on vesicle fusion, αSyn might further facilitate release of extracellular traps. To define the role of αSyn in granulocytes we differentiated human myeloid leukemia HL-60 cells into mature polynuclear granular leukocytes (dHL-60). dHL-60 granulocytes do not express αSyn [Rincón et al. BMC Genomics 19, 1-17 (2018).] but by transducing HL-60 cells with lentiviral vectors expressing fluorescently tagged aSyn, we can assess the effect of different αSyn expression levels on NET function. We generated two stably expressing aSyn-YFP cell lines, aSyn-YFP low expressing cells and aSyn-YFP high expressing cells with a twofold increase in aSyn-YFP expression. As a control, we used non-transduced WT dHL-60 cells that lack αSyn expression. Via live imaging, cells exposed to UPEC (10:1 MOI) in a culture dish were imaged with regular intervals (Fig. 3f). We observe a significant increase in extracellular DAPI signal, indicative of NETs, and within these NETs corresponding levels of aSyn-YFP (Fig. 3g, h). Together, these findings show that αSyn is released from infected neutrophils.

### Example 4. Aggregated αSyn propagates from the urinary bladder

The propagation of pathologic αSyn has been suggested to be an important mechanism in the development and the progression of synucleinopathies [Peng et al. Nat. Rev. Neurol. 16, 199-121 (2020)]. Since we discovered that innate immune cells release αSyn and the observation of aggregated αSyn in urinary bladder tissue, we examined if the triggering of αSyn aggregation could cause propagation of pathology in the nervous system.

aSyn-related pathogenicity is strongly influenced by αSyn fibrils with different biological and pathological activities that can uniquely impact phenotypic outcomes [Peelaerts et al. Nature 522, 340-344 (2015)]. We generated disease-related αSyn assemblies, by purifying and amplifying αSyn fibrils from human MSA brain. The anterior cingulate cortex of MSA patients and age-matched controls was homogenized and centrifuged on a sucrose gradient (Fig. 9a). Isolated αSyn seeds from the 10% sucrose fraction amplified successfully and reproducibly to yield αSyn fibrils that are specific to MSA and that are structurally different compared to fibrils formed by recombinant protein only (Fig 4a,b and Fig. 9 b-k). Via a protein misfolding and cyclic amplification (PMCA) assay, we amplified MSA-derived seeds free of residual brain material (Fig. 9g).

In addition to αSyn strain pathogenicity, the propagation of αSyn in MSA is dependent on environmental conditions that require the expression of oligodendroglial αSyn. Expression of αSyn in oligodendrocytes is generally low but is increased in oligodendrocytes during acute insults or demyelinating conditions. In MSA patients, glial inclusions are apparent in cervical spinal cord white matter columns including the pyramidal fibers, spinocerebellar fibers and anterolateral fibers surrounding the anterior horn [Brettschneider et al. J Neuropathol Exp Neurol. 77, 1005-1016 (2018)]. Inclusions are generally absent in the dorsal columns and neurons of the anterior and dorsal horn of MSA patients [Brettschneider et al. Neuropathol. Appl. Neurobiol. 43, 315-329 (2016)]. The spinocerebellar and spino-olivary tracts are amongst the earliest regions to be affected in MSA and connect the urogenital nerves and the spinal cord to the midbrain and cerebellum through which peripheral αSyn seeds could template and spread. To provide a permissive environment in which propagation of αSyn pathology can occur, we performed neonatal intraventricular (IVC) injection with rAAV2/9-MAG-aSyn viral vectors in transgenic human αSyn mice (hu-aSyn mice). Hu-aSyn mice do not express rodent aSyn, but only express human αSyn under control of the endogenous human αSyn promoter, at expression levels comparable to those seen for mouse αSyn in normal mice [Brettschneider et al. Neuropathol. Appl. Neurobiol. 43, 315-329 (2016)]. Humanized αSyn mice also express low levels of soluble αSyn in oligodendrocytes of spinal cord white matter columns at early age (Fig. 4). ICV injection of rAAV2/9-MAG-aSyn in hu-aSyn mice (from here on referred to as ODC-aSyn mice) results in the early and additional expression of αSyn in oligodendrocytes in the spinothalamic, spinocerebellar and spino-olivary tracts of the spinal cord at 8 weeks (Fig. 4 and Fig. 11 b-e). These ODC-αSyn mice now represent a condition with a permissive environment in which MSA fibrils could propagate.

Urinary frequency and incontinence are key features in MSAwith a progressive worsening of gait and other motor deficits that develop shortly after [Ito et al. Mov. Disord. 21, 816-823 (2006)]. Therefore, the sequential involvement of urogenital and behavioral features could represent a subtype of MSA. By injecting 5 µg of MSA fibrils or αSyn monomers as a control next to the suburothelial plexus of both sides of the urinary bladder detrusor muscle, we could test if peripheral αSyn aggregates in the urinary tract can trigger CNS pathology in the spinal cord akin to that seen in some cases of MSA. In addition, by injecting MSA fibrils in the two animal models (hu-aSyn and ODC-aSyn mice) we could define if a permissive environment (elevated levels αSyn expression) would promote propagation of αSyn pathology from the urogenital tract. After urinary bladder injection of MSA fibrils or αSyn monomers at 8 weeks of age (Fig. 4d,e), the mice were followed up to 9 months. We examined spontaneous voiding via void spot analysis on freely moving animals [Yu et al. Am. J. Physiol. - Ren. Physiol. 306, 1296-1307 (2014)] and observed that ODC-aSyn mice injected with MSA strains had different urinary voiding behavior at 9 months post injection (Fig. 4f). They had a larger voiding volume compared to animals injected with monomers or any of the other experimental conditions tested, suggestive of an abnormal change in urinary bladder function. We did not observe any difference in voiding spot frequency (data not shown). We also observed locomotor deficits. Thus, ODC-aSyn mice injected with MSA fibrils exhibited impaired gait with postural instability (Fig. 4g) and loss of fine motor control of left and right paws (Fig. 4h,i and Fig.12 a-d) by 9 months (but not at 6, months, data not shown) after fibril injection. These deficits were not apparent in mice injected with monomers or in hu-aSyn animals injected with fibrils or monomers. Collectively, our results suggest that peripheral seeding with αSyn aggregates in the permissive environment caused by αSyn overexpression in oligodendrocytes (ODC-aSyn mice) leads both to urinary bladder dysfunction and motor deficits.

To map histopathological changes associated with the observed behavioral deficits, we performed PSer-129 αSyn immunohistochemistry in sections through the spinal cord. ODC-aSyn mice injected with MSA fibrils exhibited increased αSyn pathology in white matter cervical and thoracic spinal columns compared to ODC-aSyn mice injected with αSyn monomers (Fig. 4j, k and Fig. 12 e-i). Notwithstanding the significant effect of the MSA fibrils on neuropathology, at the 11 month survival time point we also found some PSer-129 aSyn-immunopositive staining in mice transduced with the ODC-aSyn viral vector and subsequently injected with monomers (Fig. 4k). PSer-129 αSyn immunoreactivity was absent in the anterior spinal horn and in the lumbar and sacral spinal cord columns in all groups of mice. At the cervical and thoracic spinal cord levels of ODC-aSyn mice injected with MSA fibrils, the PSer-129 αSyn related changes were accompanied by morphological signs of microglial activation exclusively in the dorsal column, as detected using Iba1-immunostaining. Such microglial changes were absent in all other conditions (Fig. 41 and Fig. 13a-i). We did not observe any evidence of astrogliosis (using glial fibrillary acid protein, GFAP, immunohistochemistry) in any of the conditions tested (Fig. 13j-I). Taken together, our neuropathology studies demonstrate that αSyn aggregates, with concomitant signs of neuroinflammation, progressively develop in the dorsal spinal cord (ascending sensory pathways) in mice overexpressing αSyn in oligodendrocytes that had been injected with MSA fibrils in the urinary bladder.

### Example 5. MATERIALS AND METHODS

### Animals

We utilized 7- to 8-week-old C57BL6J or C57BL6N wild type (WT) animals that were bred in the vivarium at the Van Andel Institute for infections with UPEC. For MSA fibril injections, we used transgenic human αSyn (hu-aSyn) mice obtained from Jackson Laboratories that express the full human αSyn sequence, including the human αSyn promoter (Tg (SNCA)1Nbm) or Snca⁻; PAC-Tg (SNCA^{WT}), stock No 010710. Transgenic hu-aSyn mice were P0 for intracerebroventricular viral vector injection and 8 weeks old for fibril injection. Mice were housed at a maximum of 4 per cage under a 12-hour light-/dark cycle with ad libitum access to water and food. The housing of the animals and all procedures were carried out in accordance with the Guide for the Care and Use of Laboratory Animals (United States National Institutes of Health) and were approved by the Van Andel Institute's Institutional Animal Care and Use Committee (IACUC, AUP 17-11-019)

### Patients

Paraffin-embedded urinary bladder tissue from MSA subjects and age-matched control subjects were obtained from the Banner Sun Health Research Institute, Sun City, AZ. All MSA cases were examined and confirmed for oligodendroglial αSyn pathology. Paraffin-embedded cystitis cases and age-matched control cases were obtained from the Pathology and Biorepository Core at the Van Andel Institute, Grand Rapids, MI. Tissue used in this study was collected with the informed consent of the patients. Protocols were reviewed and approved by the Banner Health and Van Andel Insitute Institutional Review boards.

### Live imaging

HL-60 cells were cultured in suspension in RPMI 1640 medium with 20% fetal bovine serum (FBS) at 50 I.U./mL. Cells were cultured in T-75 Corning flasks with vented caps (Cat. No. 431080) and stored at 37°C in 5% CO₂. Confluency of the cells was maintained between 500,000 to 1,000,000 cells/mL. Cell counts were accomplished using trypan blue on a Bio-Rad automated cell counter T-10. Media was changed every three days by transferring the culture to an Eppendorf tube, spinning the cells down at 300xg for 4 minutes, and resuspending cells in new media. Cells were differentiated by culturing cells in media with 1 µM all-trans retinoic acid (ATRA) dissolved in DMSO for 5 days. The aSyn-YFP-expressing HL-60 cell were generated by stable lentiviral transduction of HL-60 cells with LV CMV-aSyn-YFP at three different doses. Cells were FACS-sorted into low and high aSyn-YFP expressing cells, based on YFP intensity. After differentiation with ATRA for 5 days, we challenged HL-60 WT, low-αSyn and high-aSyn with UPEC at a ratio of 10 UPEC to 1 HL-60 cell and imaged for 400 minutes using a Carl Zeiss Celldiscoverer 7, taking a pictures every 15 minutes of a 6 by 6 field. The 6 by 6 field was captured using a 20X objective and was digitally amplified by 2X. The videos were run at a speed of 2 frames per second.

### UPEC infections

The UPEC isolates used in this study were the human isolates UTI89 and its kanamycin resistant isolate UTI89 attHK022::COMGFP (kanamycin-resistant UPEC). Strains were picked with an inoculation loop from a frozen glycerol stock and cultured statically for a first overnight passage at 37°C in 20 mL Luria-Bertani broth (LB). UPEC were subsequently subcultured 1:1000 in fresh 20 ml LB statically for 18 hours to induce type 1 pilus expression. The resulting cultures were centrifuged at 3,200xg for 12 minutes, resuspended in 10 mL sterile PBS and diluted to approximately 2×10⁹ colony forming units (CFU)/mL (OD₆₀₀ = 0.22) or 1×10⁸ CFU/50µL for infection. Except for dose-response experiments all cultures were diluted to 1×10⁸ CFU per 50 µL of inoculum. For dose response applications, UPEC was diluted to 1×10⁸ or 1×10⁷ CFU per 50 µL or concentrated to 1×10⁹ CFU per 50 µL inoculum. Bacteria were inoculated into the urinary bladder of female mice via transurethral catheterization under isoflurane anesthesia as previously described [Hannan et al. PLoS Pathog. 6, e1001042--19 (2010)]. To monitor infection outcome and urinary bladder bacterial infectious titer, urinary bladders were aseptically isolated at the indicated time point and homogenized with a tissue homogenizer (Omni tissue homogenizer, TH) for 20 seconds in 1 mL sterile PBS. Homogenates were serially diluted in PBS and 50 µL of each dilution was spotted on LB agar plates to assess for total number of CFU from urinary bladder homogenates after infection.

### Viral vector production

Recombinant adeno-associated viral (rAAV) vector were generated as described previously [Van der Perren et al. J. Vis. Exp. 1-8 (2016)]. Briefly, production plasmids include the rAAV 2/9 serotype construct, the AAV transfer plasmid encoding human αSyn under the control of the oligodendroglial specific promoter myelin-associated glycoprotein (MAG) and the pAdvDeltaF6 adenoviral helper plasmid. The MAG promoter was generated by amplification of the MAG promoter from HeLa cells (Table 3). rAAV2/9-MAG αSyn was produced in Hyperflasks seeded with HEK293T cells (ATCC, Manassas, VA, USA) in Opti-MEM (Invitrogen, Merelbeke, Belgium) without addition of serum. The supernatant was collected three days after producer cell transfection and concentrated with tangential flow filtration. Viral vectors were subsequently concentrated via iodixanol step gradient and centrifugation at 27,000xg for two hours. Gradient fractions were collected and pooled between refraction index 1.39 and 1.42 after which a final centrifugation and concentration step was performed with Vivaspin 6 columns (PES, 100,000 MWCO, Sartorius AG, Goettingen, Germany) at 3,000xg. Samples were aliquoted and stored at -80°C for further use. Viral titers were determined via real-time PCR using a primer probe set for polyA (Table 3) to assess genomic copies (GCs) and were approximately 6×10¹² GC/mL.

**Table 3. Primers used for viral vector development and characterization**

| Primer | Sequence |
|---|---|
| MAG sense | 5′-TATATGCTAGC CCTCAGAAGG AACCAACACT GCCAGCACT T-3′ |
| MAG antisense | 5′-ATTTATGATA TCGCCCCCAC TTGCCAGCCC CTCCCCTCCC- 3′ |
| polyA sense | 5'-TCTAGTTGCC AGCCATCTGT TGT-3' |
| polyA antisense | 5'-TGGGAGTGGC ACCTTCCA-3' |
| polyA probe | 5'-TCCCCCGTGC CTTCCTTGACC-3 |

### Intracerebroventricular injections

Newborn pups were injected intracerebroventricularly (ICV) at P0 (6-12 hours after birth) and when milk spots were visible. Only half of the pups were taken from their mother during nursing. Before surgery, pups were cryoanesthetized on a metal plate on ice to cool down body temperature to 4°C and subsequently transferred to a cooled neonatal surgery frame that was maintained at 2-6°C with dry ice. The head was wiped with 70% ethanol, leveled parallel with the injection frame and fixed gently between two ear bars. Cranial blood vessels were used to determine the lambda sutures for ICV injection coordinates. A 34G 10 µl Hamilton needle (Hamilton, Reno, NV, USA) was placed at A/P: +0.165 cm and M/L: ±0.080 cm from lambda. The bevel of the needle was used to pierce the skull and positioned just below the flattened skull after which it was lowered to D/V: -0.170 cm and retracted to D/V: -0.130 cm for ventricular injection. Injection was performed bilaterally for a volume of 2 µL for each ventricle (for a total volume of 4 µL) at a perfusion rate of 0.5 µL/minute and a total titer of approximately 2×10¹⁰ GC. After the injection, the skull was flattened by gently retracting the needle to open the ventricles and the needle was left for another 2 minutes to allow the injected bolus to spread throughout the ventricles. Viral vector was supplemented with 0.5% trypan blue dye to achieve a 0.05% trypan blue solution in order to assess successful ventricular injection. After injection, pups were allowed to recover on a 37°C heating pad and returned to their home cage. The procedures as described above for IVC surgery were approved by the Van Andel Institute Institutional Animal Care and Use Committee (IACUC).

### Human disease brain tissue for fibril amplification

Frozen brain tissue from the anterior cingulate was obtained from subjects with MSA, and age-matched control subjects from the Michigan Brain Bank (University of Michigan, Ann Arbor, MI, USA). Brain tissue was collected with the informed consent of the patients. Protocols were approved by the Institutional Review Board of the University of Michigan and abide by the Declaration of Helsinki principles. Samples were examined at autopsy by neuropathologists for diagnosis.

### Brain tissue processing

Total of 600 µL lysis buffer (1xPBS, 2mM EGTA, 2mM EDTA, PhosSTOP^{™} (cat# 4906845001, Sigma-Aldrich, Burlington, MA, USA), cOmplete^{™} EDTA-free Protease Inhibitor Cocktail mini (cat# 11836170001, Sigma-Aldrich), 6 µL /mL saturated phenylmethylsulfonyl fluoride (PMSF), and 1mM sodium azide in PBS) was added to 0.5 g of brain tissue (anterior cingulate) of individuals diagnosed with MSA, and age-matched controls without αSyn pathology (Table 4).

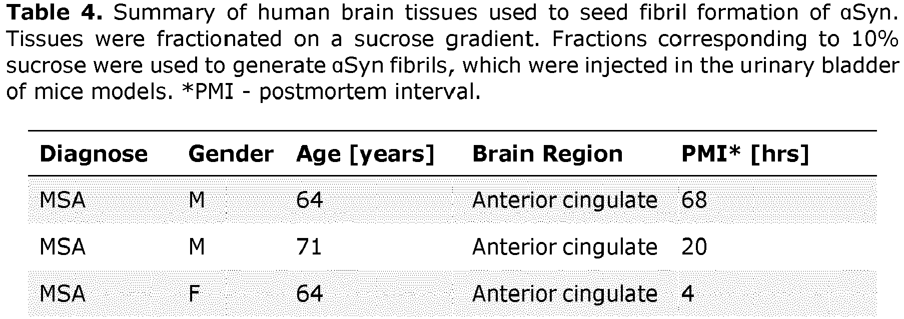

Tissue samples were homogenized with 3.2 mm stainless steel beads three times for 1 minutes (speed 4) with cooling on ice for 5 minutes in between processing (Nova Advance homogenizer, Next Advance, Troy, NY, USA). Then 300 µL of lysis buffer was added, and samples were homogenized one more time for 2 minutes.

Homogenized samples were centrifuged at 1,000xg for 10 minutes. Then the supernatant was additionally centrifuged at 18,000xg for 10 minutes. TritonX-100 was added to the resulting supernatant to make a 2% final concentration. After incubation with rocking for 30 minutes at 4°C, samples were centrifuged at 18,000xg for 10 minutes. Then the supernatant was loaded on the top of a sucrose gradient. Gradients were prepared by layering 40% (w/v), 30% (w/v), 20% (w/v), 15% (w/v), 10% (w/v), and 5% (w/v) sucrose in PBS. Starting with the highest concentration, sucrose solutions were added one at a time followed by flash freezing in a liquid nitrogen. Gradients were centrifuged at 200,000xg (SW 41Ti, Beckman Coulter) for 16 hours at 4°C. Gradient layers corresponding to different sucrose concentrations were pooled and consequently used to seed fibril formation of recombinant αSyn.

### Recombinant protein preparation

Recombinantly expressed full-length αSyn was prepared as previously described. Briefly, E. coli cell cultures were grown in LB media containing ampicillin and induced with 0.5 mM isopropyl β-D-1-thiogalactopyranoside for 3 hours. Cells were harvested by centrifugation, the cell pellet then resuspended in lysis buffer (200 mM Tris-HCl pH 8.0, cOmplete^{™} EDTA-free Protease Inhibitor Cocktail mini (cat# 11836170001, Sigma-Aldrich/Roche, Basel, Switzerland), and 6 µL/mL saturated PMSF) and cells were lysed using an Emulsiflex homogenizer (Avestin, Ottawa, Canada). After centrifugation for 15 minutes at 32,000g, 0.23 g/mL ammonium sulfate was added to the supernatant. After incubation for 20 minutes, samples were centrifuged 32,000×g for 20 minutes. The pellet was resuspended in 20 mM Tris, pH 8.0 (Buffer A), and dialyzed against 4 L of buffer A, which was exchanged twice. The next day, samples were loaded onto a 5 mL Q-Sepharose FF column (GE Healthcare) equilibrated with Buffer A and eluted against a linear gradient of Buffer B (1M NaCl, 20 mM Tris-HCl, pH 8.0, pH 8.0). Fractions containing αSyn were identified using SDS-PAGE, collected, and further purified by pushing the protein through 50 kDa Amicon Ultra-15 Centrifugal Filter Unit (Millipore Sigma, Burlington, MA). The flow through was further concentrated and assessed by SDS-PAGE. Endotoxins were removed by incubation of purified αSyn with Pierce High-Capacity Endotoxin Removal Resin (Cat. # 88270, Fisher), as specified by the manufacturer.

### Fibril formation

Fibril assays were carried out with 100 µM αSyn in a sample buffer containing 150 mM NaCl, 25 mM sodium phosphate pH 7.5, and 1mM soidium azide. Prior to the assay recombinant αSyn was filtered through a 0.05 µm pore filter. The first generation of fibrils was formed by adding 2% w/w (protein weight) of fractionated brain MSA tissue pulled from 10% sucrose gradient. The progeny fibrils (second, third, fourth, etc generations) were produced by adding 15% w/w (protein weight) of sonicated parent fibrils to the monomeric αSyn. Volumes of 60 µL were dispensed in each well of 384-well glass bottom plates (Greinier, Monroe, NC, USA, cat# 781892). Plates were then incubated at 37°C in FLUOstar Omega (BMG Labtech Inc, Ortenburg, Germany) by shaking. Three wells were supplemented with 10 µM ThioflavinT (ThT) to monitor the progress of fibril formation. Fluorescence was measured with gain set at 90%, an excitation wavelength of 440 nm and emission wavelength of 490 nm. At reaction completion (ThT fluorescence reached plateau), samples from all wells, except for the ones containing ThT, were combined.

### Preparation of fibrils for animal injection

Fibrils of αSyn were washed to remove unpolymerized protein. After spinning 1mL of sample at 17,000×g for 5 minutes, the pelleted fibrils were resuspended with a sample buffer without sodium azide. Sample spinning/resuspension was repeated twice. The last pellet was resuspended with a 200 µL sample buffer without sodium azide, resulting in 5x concentrated fibrils. After testing for endotoxin using (Pierce LAL Chromogenic Endotoxin Quantitation Kit, cat# 88282), fibrils were sonicated. The concentration of αSyn (monomer) was determined by dilution of the sample 5x with 6M guanidinium hydrochloride, followed by boiling for 1 minutes. After cooling, protein concentration was determined by measuring A₂₈₀ with an extinction coefficient of 5960 M⁻¹ cm⁻¹. Samples (350 µM monomeric αSyn) were flash frozen in 25 µL aliquots on a liquid nitrogen and stored at -80°C until use.

### Sonication

If specified in the test, fibrils were sonicated (PIP 50, DF 10%, and CPB 200) for 120-160 cycles (1 sec ON and 1 sec OFF) in tubes with disposable probes (microTUBE-130 AFA Fiber Screw-Cap, cat# PN 520216, Covaris, Woburn, MA, USA) in an M220 Focused-ultrasonicator (Covaris).

### Transmission electron microscopy (TEM)

Negatively stained specimens for TEM were prepared by applying 5 µL of sample to hydrophilic 400 mesh carbon-coated Formvar support films mounted on copper grids (Ted Pella, Inc., Redding, CA, USA, cat# 01702-F). The samples were allowed to adhere for 4 minutes, rinsed twice with distilled water, and stained for 60-90 seconds with 5 µL of 1% uranyl acetate (Ted Pella, Inc.). All samples were imaged at an accelerating voltage of 80 kV in a JEM-1400 (JOEL).

### Urinary bladder recombinant protein injections

Male and female transgenic 8-week old transgenic hu-aSyn animals were anesthetized with a mixture of oxygen and isoflurane. After disinfection of the lower abdominal region, a small incision was made above the urinary bladder after which the urinary bladder was isolated and fixed for injection. Mice were inoculated with 1.5 µL of MSA fibrils or αSyn monomers at equivalent concentrations of 250 µM for a total of 3 µg in two opposite spots of the lower detrusor muscle adjacent to the urinary tract. Amplified fibrils from 3 MSA patients were mixed and injected as one bolus. Each injection was performed with a microinjector and confirmed with 0.05% toluidine blue to visually assess correct injection and spreading of the injected material throughout the urinary bladder tissue. After injection, the lower abdominal peritoneum and skin were sutured and disinfected again. Animals were allowed to recover on a heating pad at 37°C and monitored during recovery.

### Behavioral analysis

To assess urinary bladder function and voiding, the spontaneous void spot assay was conducted on freely moving animals, as described previously [Yu et al. cited above]. Mice were transferred to a clean, empty cage with a Whatman Grade 540 hardened Ashless filter paper covering the cage bottom with nestlet enrichment placed on top of the membrane. One mouse was housed per cage during the voiding analysis. The assay was conducted over a 4-hour period on male mice in the morning from 9 AM until 1 PM. Animals had access to food but not to water and were tested in the same location as they were normally housed. Filter papers were imaged with UV light and analyzed with Fiji software (1.46). A standard was calculated to measure total voiding volume and voiding spot frequency.

Gait analysis was performed using the Catwalk XT automated gait analysis system that consists of a 130 × 10 cm glass-floor walkway with fluorescence light beaming in the glass to illuminates the animal's paws (Noldus Information Technology). Each experiment was performed according to the manufacturer's instructions. Briefly, mice were acclimatized for 1 hour in the behavior room with ambient red lighting. To encourage runs across the catwalk the home cage of each mice was used as cue. The pawprint detection settings were determined automatically by the catwalk XT dedicated software (ver. 10.6) by tracking a control mouse with a weight representable of the cohort. All mice were tested in 3 consecutive runs over 3 consecutive days at 6- and 9 months post inoculation with MSA fibrils or monomeric αSyn protein. Compliant runs were defined as lasting between 1-5 seconds with a maximum speed variation of 60% [Caballero-Garrido et al. Behav. Brain Res. 331, 282-296 (2017)]. If a subject failed to run across the catwalk, a run was repeated until compliant. In rare instances a mouse completed <3 compliant runs. All runs were recorded and pawprints labeled automatically. Acquired data was segmented according to strict inclusion criteria including a minimum number of steps per run of 10, average speed ranging from 1 to 50 cm/seconds while all non-compliant or partially characterized runs were excluded. Accordingly, 81.6 ±6% of acquired runs were included for further data analysis and graphic representation (1177/1457 runs).

### Euthanasia and tissue collection

Animals were anesthetized with sodium pentobarbital (130 mg/kg; Sigma Aldrich) for tissue collection at the indicated time points. Urinary bladders were isolated prior to perfusion and homogenized or fixed with 4% PFA with overnight fixation for subsequent protein analysis or histological analysis, respectively. For animals injected with recombinant protein, perfusion was performed at the 9-month time point (when animals were approximately 11 months old) with isotonic saline. The urinary bladder was isolated before perfusion and was fixed via 4% PFA overnight fixation. Spinal cord and brain were isolated after transcardial perfusion with saline and fixation with 4% PFA. Both spinal cord and brain were post fixed overnight with 4% PFA and then stored at 4°C in 30% sucrose in phosphate buffer until sectioning.

### Western blotting

Urinary bladders were isolated from infected animals in 1 mL sterile PBS supplemented with protease and phosphatase inhibitor (Halt Protease Inhibitor Cocktail 100x, ThermoFisher) and homogenized using a tissue homogenizer (Omni tissue homogenizer) for 20 seconds. Samples were spun at 6,000xg at 4°C for 5 minutes to remove cell debris. The supernatant was heated at 95°C for 10 minutes with Laemmli buffer and stored at -80°C for downstream analysis. For detection of aggregated aSyn, urinary bladders were homogenized in sterile PBS with protease and phosphatase inhibitor. Homogenized tissue was treated with 1% sarkosyl for 60 minutes at room temperature while gently rotating. Denatured samples were spun at 6,000xg for 10 minutes at 20°C to remove remaining cell debris and the supernatant was used for isolation of aggregated protein. Sarkosyl samples were ultracentrifuged at 100,000xg for 60 minutes at 20°C. The resulting pellet was washed with PBS and resolubilized in PBS with 1% sarkosyl for a second round of ultracentrifugation for 20 minutes at 20°C. The pellet was washed in Laemmli buffer containing 2% SDS to disaggregate αSyn ssemblies and denatured at 95°C for 10 minutes. Samples were stored at -80°C for downstream analysis. Samples were separated via SDS-PAGE using a 4-15% Criterion TGX precast gels (Bio-Rad) and transferred using the Trans-blot Turbo system (Biorad) to a PVDF membrane. Membrane blocking was performed with 5% BSA in 0.1% triton-X in PBS for 30 minutes whereafter primary antibody was applied according to the concentration listed in Table 5 for O/N incubation at 4°C in 5% BSA in 0.1% triton-X in PBS. Membranes were washed three times next day with 0.1% triton-X in PBS, and incubated with HRP-labeled secondary antibody (Table 5) for detection with Clarity Western ECL Substrate (Bio-Rad, 1705061). Chemiluminescent signal was detected using Biorad ChemiDoc and ImageJ software.

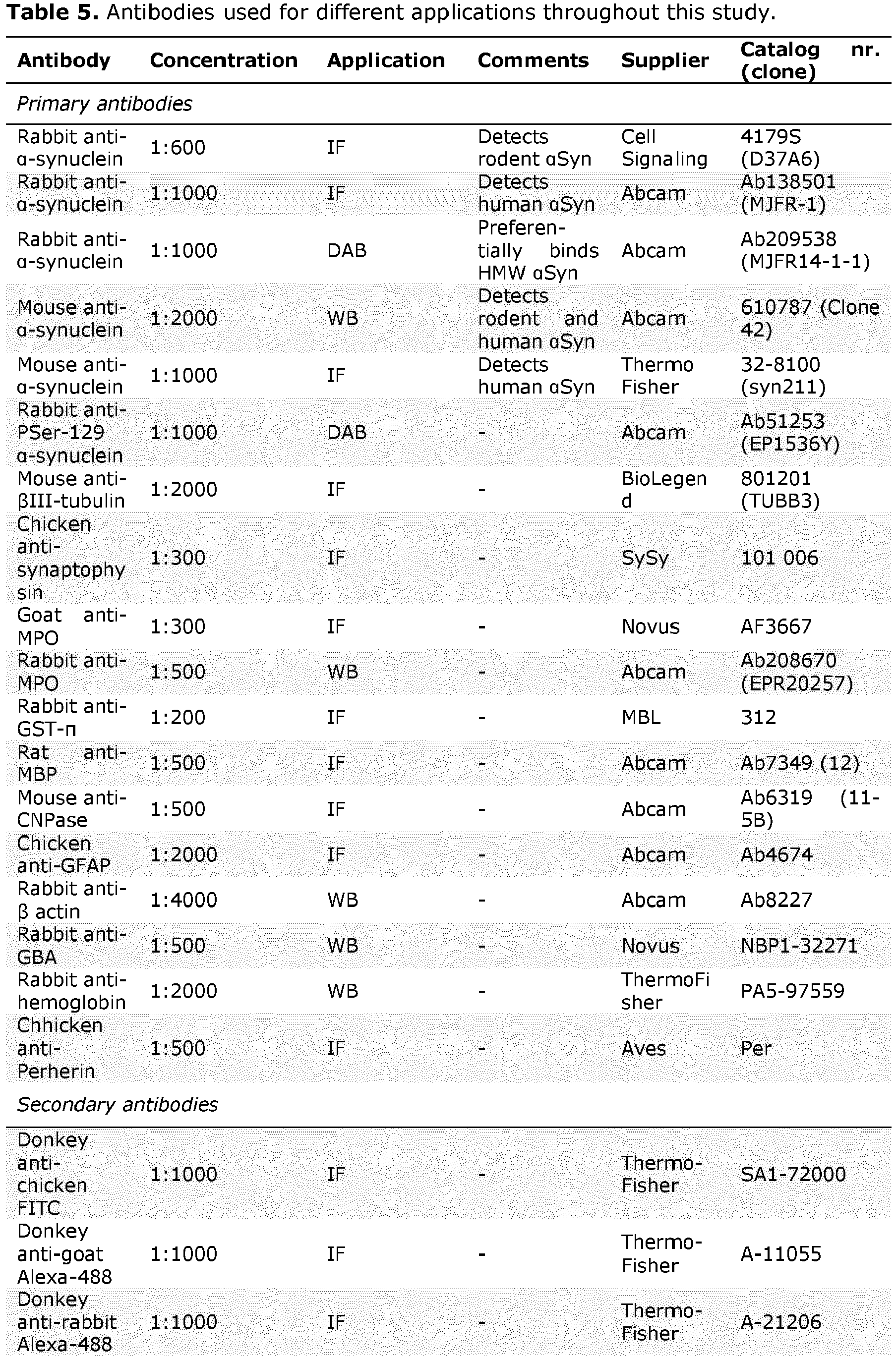

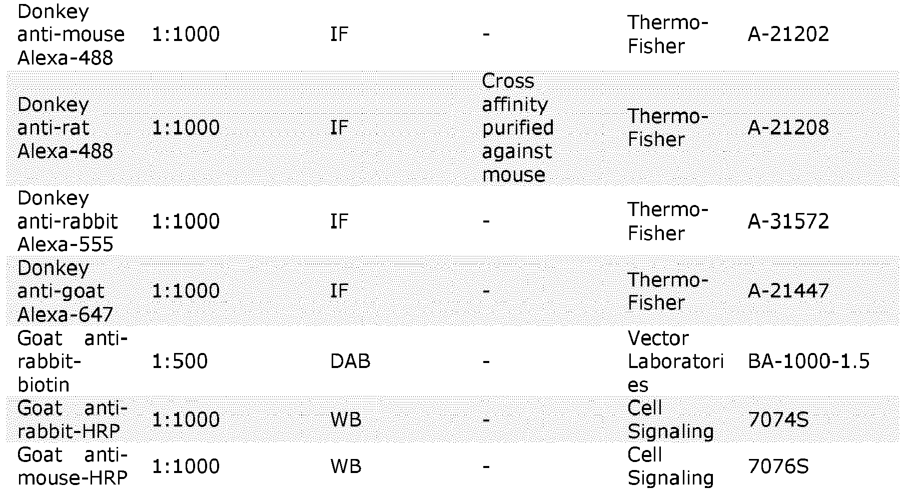

### Immunohistological analysis

Urinary bladder tissue was embedded in paraffin and sectioned at 6 µm. Urinary bladder tissues were stained on glass mounting slides with 1 mL of incubation solution. Slides were heated at 60°C on a heating block for 15 min and rehydrated via serial rehydration in xylene, 100% ethanol, 90% ethanol and 70% ethanol and distilled water. Antigen retrieval for urinary bladder was performed on paraffin-embedded urinary bladder tissue with the universal HIER antigen retrieval reagent (Abcam) for 30 minutes in a steam cooker at 95-100°C. For immunofluorescent analysis, sections were blocked with 10% donkey serum (Millipore-Sigma) in 0.1% Triton-X in PBS for 30 min at room temperature. For antigen detection, different concentrations of primary antibodies are listed in Table 5 and were used at 4°C overnight. Sections were triple washed in 0.1% Triton-X in PBS and incubated with secondary antibody (Table 5) and 1:1000 DAPI for two hours at room temperature after which the sections were washed again with 0.1% Triton-X in PBS. Urinary bladder tissues were treated with Trueblack Lipofuscin Autofluorescent Quencher (Biotium) for 3 minutes to quench non-specific extracellular fluorescence and cellular autofluorescence. Slides were sealed with Vectashield Antifade Mounting Medium (Vector Laboratories). Images were acquired using an A1plus-RSi Scanning Confocal microscope (Nikon). For DAB immunoprecipitation and antigen detection, samples were blocked with 10% goat serum for 1 h after which they were incubated with primary antibody listed in Table 5 at 4°C overnight. Next day, slides were triple washed with 0.1% Triton-X in PBS and incubated with biotinylated anti-rabbit antibody (Vector Laboratories, BA-1000-1.5) for 2 hours at RT, triple washed again and treated with Vectastain ABC kit (Vector Laboratories, PK-4000). Antigen detection was performed with Vector DAB (Vector Laboratories, SK-4100). After dehydration, slides were coverslipped with Cytoseal 60 mounting medium (Thermo Fisher Scientific). The sections were viewed under a Nikon Eclipse Ni-U microscope (Nikon); images were captured with a color Retiga Exi digital CCD camera (QImaging) using NIS Elements AR 4.00.08 software (Nikon)

Brain and spinal cord tissue were frozen and collected as a series of coronal sections of 40 µm via microtome sectioning (Leica). For immunofluorescent analysis, single brain or spinal sections or a series of free-floating tissue sections (every 240 µm) were blocked with 10% donkey or goat serum (Millipore) and 4% bovine serum albumin in 0.1% Triton-X in PBS for 2 h at room temperature. Samples were incubated with primary antibody listed in Table 5 at room temperature overnight. Next day, slides were triple washed with 0.1% Triton-X in PBS and incubated with secondary antibody (Table 5) for two hours at room temperature after which the sections were washed again with 0.1% Triton-X in PBS. Slides were sealed with Vectashield Antifade Mounting Medium with DAPI (Vector Laboratories). The sections were viewed under a Nikon Eclipse Ni-U microscope (Nikon); images were captured with a color Retiga Exi digital CCD camera (QImaging) using NIS Elements AR 4.00.08 software (Nikon) or a confocal microscope (Nikon A1plus-RSi Laser Scanning Confocal Microscope). For immunohistochemistry, a series of free-floating tissue sections were stained using a primary antibody listed in Table 5 and biotinylated secondary sera at 1:500 (Table5). For the detection of the antibody with DAB, we used a standard peroxidase-based method (Vectastain ABC kit and DAB kit; Vector Laboratories). After dehydration, slides were coverslipped with Cytoseal 60 mounting medium (Thermo Fisher Scientific). Slides were scanned using an Aperio AT2 scanner (Leica).

### Statistical analysis

Statistical analysis was performed using GraphPad Prism 8 software. The type of analysis with post hoc correction for multiple testing is indicated in the legend of each figure. Statistical levels were set at ^{∗}p <0.05, ^{∗∗}p<0.01, ^{∗∗∗}p<0.001.

## Claims

1. A non-therapeutic method of screening a candidate compound for use as a drug for the treatment or prevention of a demyelinating disease, comprising the steps of:
a) Providing a transgenic rodent wherein the expression of wild type rodent alpha synuclein is inactivated and carrying a transgenic construct expressing human alpha synuclein in neurons and oligodendrocytes,
b) delivering to the transgenic rodent of step a candidate compound,
c) determining the expression of alpha synuclein in oligodendrocytes and/or of the rodent, and comparing with the expression in a control rodent as described in step a) which did not receive the candidate compound,
wherein a decrease in alpha synuclein expressed in oligodendrocytes compared to the control rodent is indicative of a therapeutic activity of the candidate compound against the demyelinating disease.

2. The method according to claim 1, wherein the demyelinating disease is Multiple System Atrophy (MSA).

3. The method according to claim 1 or 2, wherein the rodent is sacrificed prior to determining the expression of alpha synuclein in step c).

4. The method according to any one of claims 1 to 3, wherein the expression of wild type rodent alpha synuclein is inactivated by deletion of the wild type alpha synuclein gene.

5. The method according to any one of claims 1 to 4, further determining the expression of human alpha synuclein in the central and/or peripheral nervous system, wherein a decrease in alpha synuclein expressed in central and/or peripheral nervous system compared to the control rodent is indicative of a therapeutic activity of the test compound against the demyelinating disease.

6. The method according to any one of claims 1 to 5, further delivering to the rodent animal human alpha synuclein via viral vectors and/or injection of alpha synuclein fibrils.

7. The method according to any one of claims 1 to 6, wherein the test compound is administered prior to the development of MSA pathology.

8. The method according to any one of claims 1 to 7, wherein the test compound is administered to a rodent showing MSA pathology.

9. The method according to any one of claims 1 to 8, wherein the expression of the human alpha synuclein is under the control of human alpha synuclein promotor.

10. The method according to any one of claims 1 to 9, wherein the human alpha synuclein is wild type alpha synuclein.

11. The method according to any one of claims 1 to 10, wherein the test compound is a recombinant Adeno Associated viral vector comprising miRNA against alpha synuclein.
